# EUROPEAN PATENT SPECIFICATION

(11) **EP 3 932 491 B1**
(45) Date of publication and mention of the grant of the patent: **26.08.2026**
(21) Application number: 20763424.7
(22) Date of filing: 27.02.2020
(51) Int. Cl.: A61P 35/00, C07K 7/06, C07K 7/08, C12N 15/12, C12Q 1/04, C12Q 1/6886, A61K 38/08, A61K 38/10, G01N 33/48, G01N 33/49, G01N 33/50, A61K 39/00, C07K 14/47

(54) **METHOD FOR SELECTING SUBJECT LIKELY BENEFITING FROM PHARMACEUTICAL COMPOSITION FOR TREATING OR PREVENTING CANCER**
VERFAHREN ZUR AUSWAHL VON PATIENTEN, DIE WAHRSCHEINLICH VON EINER PHARMAZEUTISCHEN ZUSAMMENSETZUNG ZUR BEHANDLUNG ODER PRÄVENTION VON KREBS PROFITIEREN
PROCÉDÉ DE SÉLECTION DE SUJET SUSCEPTIBLE DE TIRER PROFIT D'UNE COMPOSITION PHARMACEUTIQUE PERMETTANT LE TRAITEMENT OU LA PRÉVENTION DU CANCER

(30) Priority: 28.02.2019 JP 2019036458
(43) Date of publication of application: 05.01.2022
(73) Proprietor: International Institute of Cancer Immunology, Inc., Osaka 564-0053 (JP)
(72) Inventor: YAMAKAWA, Erina, Osaka-shi, Osaka 554-0022 (JP); GOTO, Masashi, Osaka-shi, Osaka 541-0045 (JP)
(74) Representative: Vossius & Partner Patentanwälte Rechtsanwälte mbB
(86) International application number: PCT/JP2020/008180
(87) International publication number: WO 2020/175657

(56) References cited:
- WO-A1-2014/157692
- CARTER J H ET AL: "Evidence for WT1 as a potential target for immunotherapy of lethal ovarian cancer", vol. 77, no. 13, Supplement 1, 1 July 2017 (2017-07-01), XP009540028, ISSN: 1538-7445, Retrieved from the Internet <URL:https://aacrjournals.org/cancerres/article/77/13_Supplement/2793/618390/Abstract-2793-Evidence-for-WT1-as-a-potential>
- CARTER JULIA H ET AL: "Transcription factors WT1 and p53 combined: a prognostic biomarker in ovarian cancer", BRITISH JOURNAL OF CANCER, NATURE PUBLISHING GROUP UK, LONDON, vol. 119, no. 4, 30 July 2018 (2018-07-30), pages 462 - 470, XP036822816, ISSN: 0007-0920, [retrieved on 20180730], DOI: 10.1038/S41416-018-0191-X
- NAKAE YOSHIKI ET AL: "Two distinct effector memory cell populations of WT1 (Wilms' tumor gene 1)-specific cytotoxic T lymphocytes in acute myeloid leukemia patients", vol. 64, no. 7, 3 April 2015 (2015-04-03), Berlin/Heidelberg, pages 791 - 804, XP055973737, ISSN: 0340-7004, Retrieved from the Internet <URL:https://link.springer.com/content/pdf/10.1007/s00262-015-1683-7.pdf> DOI: 10.1007/s00262-015-1683-7
- SAMSTEIN ROBERT M ET AL: "Tumor mutational load predicts survival after immunotherapy across multiple cancer types", NATURE GENETICS, NATURE PUBLISHING GROUP US, NEW YORK, vol. 51, no. 2, 14 January 2019 (2019-01-14), pages 202 - 206, XP036900827, ISSN: 1061-4036, [retrieved on 20190114], DOI: 10.1038/S41588-018-0312-8
- CALIBASI-KOCAL GIZEM ET AL: "Genetic Diversity and Disease Susceptibility", 17 October 2018 (2018-10-17), XP055973749, ISBN: 978-1-78984-202-9, Retrieved from the Internet <URL:http://dx.doi.org/10.5772/intechopen.76934> DOI: 10.5772/intechopen.76934
- THOMAS, MARIAM. ET AL.: "Integration of Technical, Bioinformatic, and Variant Assessment Approaches in the Validation of a Targeted Next-Generation Sequencing Panel for Myeloid Malignancies", ARCH PATHOL LAB MED, vol. 141, 2017, pages 759 - 775, XP055734956
- HANG, AU CHUN. ET AL.: "Clinical evaluation of panel testing by next-generation sequencing (NGS) for gene mutaions in myeloid neoplasms", DIAGNOSTIC PATHOLOGY, 2016, XP055734969

## Description

### Technical Field

The present invention relates to a method for selecting a potential subject with benefiting from a pharmaceutical composition for treating or preventing cancer according to claim 1.

### Background Art

Cell-mediated immunity, in particular, cytotoxic T lymphocytes (referred to as CTL), play an important role in the elimination of tumor cells, virus-infected cells, and the like by the living body. CTL is produced through the differentiation and proliferation of precursor T cells that have recognized a complex of an antigen peptide on tumor cells (tumor antigen peptide) and an MHC (major histocompatibility complex) class I antigen, and attacks tumor cells.

MHC is called human leukocyte antigen (HLA) for humans, and HLA-A, B and Cw, etc. are known. The tumor antigen peptide is produced through the intracellular synthesis of a protein highly expressed in tumor, i.e., a tumor antigen protein, followed by intracellular degradation by protease. The produced tumor antigen peptide binds to an MHC class I antigen in the endoplasmic reticulum to form a complex, which is transported to cell surface for antigen presentation. Tumor-reactive CTL recognizes the antigen-presented tumor antigen peptide (killer peptide) and exhibits an antitumor effect via cytotoxic action or lymphokine production.

The development of treatment methods for potentiating cancer-specific CTL in the bodies of cancer patients has been studied by utilizing a tumor antigen protein or a killer peptide derived from a tumor antigen as a main component of a so-called cancer immunotherapeutic agent (cancer vaccine). For example, cancer immunotherapy targeting WT1 (Wilm's tumor 1) is in the process of being developed. WT1 is a gene that has been identified as a responsible gene of Wilms tumor, which is kidney cancer in children, and is a transcriptional factor having a zinc finger structure (see Non Patent Literature 1). The WT1 gene was originally reported to be a cancer suppressor gene, but has been found to rather work as a cancer gene in hematopoietic organ tumor or solid cancer by subsequent research. Also, it has been reported that the WT1 gene is highly expressed in many malignant tumors (see Non Patent Literature 2). WT1 is considered to be a novel tumor antigen protein in leukemia and solid cancer (see Non Patent Literature 3). Accordingly, cancer vaccine therapy or dendritic cell therapy using the WT1 protein or a peptide derived from the WT1 protein, a TCR-like antibody that recognizes an HLA complex with a peptide derived from the WT1 protein, or chimeric antigen receptor (CAR) genetically engineered T cell therapy using the TCR-like antibody, etc. is under development.

As for the WT1 protein, for example, killer peptides, such as WT1₁₂₆₋₁₃₄ peptide, WT1₂₃₅₋₂₄₃ peptide, WT1₁₀₋₁₈ peptide, WT1₁₈₇₋₁₉₅ peptide, WT1₃₀₂₋₃₁₀ peptide, and WT1₃₇₋₄₅ peptide, which are displayed by binding to MHC class I have been reported (see Patent Literature 1, Patent Literature 2, and Non Patent Literatures 4 and 5).

Examples of cells that play an important role in cancer immunotherapy include helper T (Th1) cells, in addition to CTL. In general, an antigen protein is degraded in intracellular lysosome, and some of fragment peptides constituted by amino acids on the order of 13 to 17 residues bind as antigen peptides (helper peptides) to MHC class II molecules. Then, the complex of the antigen peptide and the MHC class II molecule is presented by a TCR/CD3 complex so that activated Th1 cells promote the induction and activation of CTL. HLA-DR, DQ and DP, etc. are known as human MHC class II molecules, and a plurality of helper peptides derived from the WT1 protein have been identified so far (see Non Patent Literatures 6 and 7).

For sufficiently exerting the effect of cancer immunotherapy, it is important to predict the response of subjects in advance and select a subject that are expected to benefit therefrom. However, cancer immunotherapy targeting WT1 by using the killer peptide that is presented by binding to MHC class I and/or the helper peptide that binds to an MHC class II molecule is in the process of being developed, whereas there has been no report on a method for selecting in advance a subject benefiting from treatment or prevention with a WT1 peptide vaccine using an antigen peptide derived from a WT1 antigen protein.

### Citation List

### Patent Literature

Patent Literature 1: International Publication No. WO 00/06602
Patent Literature 2: International Publication No. WO 00/18795

### Non Patent Literature

Non Patent Literature 1: Am J Hum Genet. 1993; 52: 192-203
Non Patent Literature 2: Blood. 1997; 89: 1405-1412
Non Patent Literature 3: Immunogenetics. 2000; 51: 99-107
Non Patent Literature 4: Clin Cancer Res. 2005; 11: 8799-807
Non Patent Literature 5: Blood. 2008 Oct 1; 112 (7): 2956-64
Non Patent Literature 6: J Immunother. 2007; 30: 282-93
Non Patent Literature 7: Cancer Immunol Immunother. 2010; 59: 1467-79

Carter et al. 2017 (Cancer Res. 77(13) Suppl. 1) and Carter et al. 2018 (Br J Cancer 119(4):462-470) disclose that detection of mutated p53 can be used to select ovarian cancer patients for WT1 immunotherapy.

### Summary of Invention

### Technical Problem

Accordingly, an object of the present invention is to provide a method for selecting a potential subject with benefiting from a pharmaceutical composition for treating or preventing cancer according to claim 1. Here, the pharmaceutical composition comprises a WT1 killer peptide and optionally a WT1 helper peptide.

### Solution to Problem

The present inventors have conducted diligent studies to attain the object and consequently completed the present invention by finding a method according to claim 1 for selecting a potential subject with benefiting from a pharmaceutical composition for treating or preventing cancer on the basis of the presence or absence of a mutation in tumor protein p53 (TP53) gene and BCL6 co-repressor (BCOR) gene, and optionally the mRNA expression level of WT1 gene, etc.

### Advantageous Effects of Invention

According to the present invention, a method for selecting a potential subject with benefiting from a pharmaceutical composition for treating or preventing cancer is provided according to claim 1. Here, the pharmaceutical composition comprises a WT1 killer peptide and optionally a WT1 helper peptide, or a pharmaceutically acceptable salt thereof. By selecting a potential subject with benefiting from each pharmaceutical composition, effective treatment or prevention of cancer can be performed by using the pharmaceutical composition.

### Brief Description of Drawings

Fig. 1 shows test results of a WT1 peptide cocktail vaccine, and results of performing comparison with a control (BSC) in the ONTIME test of rigosertib.
Fig. 2 shows the survival curves of TP53 wild type and BCOR wild type, and TP53 mutant or BCOR mutant.
Fig. 3 shows results of comparing survival curves based on the positivity or negativity of WT1 antigen peptide-specific immune response as to TP53 wild type and BCOR wild type, and TP53 mutant or BCOR mutant.
Fig. 4 shows results of comparing survival curves by the expression level of WT1 mRNA in Example 5 (results (1)).
Fig. 5 shows results of comparing survival curves based on the positivity or negativity of WT1 antigen peptide-specific immune response as to the expression level of WT1 mRNA (results (1)).
Fig. 6 shows results of comparing survival curves by the expression level of WT1 mRNA in Example 5 (results (2)).
Fig. 7 shows results of comparing survival curves based on the positivity or negativity of WT1 antigen peptide-specific immune response as to the expression level of WT1 mRNA (results (2)).
Fig. 8 shows analysis results by HLA tetramer assay, and results of bi-directionally analyzing the determination of a DTH test using a WT1 killer peptide conjugate.
Fig. 9 shows results of comparing survival curves as to the positivity or negativity of WT1 antigen peptide-specific immune response, and the stabilization of myeloblasts.
Fig. 10 shows results of comparing transition periods to acute myeloid leukemia (AML) based on the positivity or negativity of WT1 antigen peptide-specific immune response.
Fig. 11 shows results of comparing survival curves based on the positivity or negativity of WT1 antigen peptide-specific immune response.
Fig. 12 shows results of comparing survival curves based on the positivity or negativity of WT1 antigen peptide-specific immune response as to the karyotype of IPSS-R.
Fig. 13 shows results of comparing the median survival time of each sex difference with historical data and the median survival time of BSC of a rigosertib test.
Fig. 14 shows results of comparing survival curves based on the expression level of WT1 mRNA in Example 11.
Fig. 15 shows results of comparing survival curves based on the positivity or negativity of WT1 antigen peptide-specific immune response as to the expression level of WT1 mRNA.
Fig. 16 shows the relationship between a WT1 mRNA expression level in peripheral blood and a WT1 mRNA expression level in bone marrow fluid.

### Description of Embodiments

Hereinafter, embodiments of the present invention will be described in detail.

In the present specification, the "amino acid residue" means a moiety corresponding to one unit of an amino acid constituting a peptide or a protein on a peptide or protein molecule. Examples of the "amino acid residue" include natural or non-natural α-amino acid residues, β-amino acid residues, γ-amino acid residues and δ-amino acid residues. Specifically, examples thereof include natural α-amino acid residues, an ornithine residue, a homoserine residue, a homocysteine residue, β-alanine, γ-aminobutanoic acid and δ-aminopentanoic acid. In the case that the "amino acid residue" may be an optically active form, an L form is preferable, though either an L form or a D form is acceptable.

In the present specification, the "amino acid residue" may be indicated by an abbreviation and is described in the following abbreviations.
Ala or A: alanine residue
Arg or R: arginine residue
Asn or N: asparagine residue
Asp or D: aspartic acid residue
Cys or C: cysteine residue
Gln or Q: glutamine residue
Glu or E: glutamic acid residue
Gly or G: glycine residue
His or H: histidine residue
Ile or I: isoleucine residue
Leu or L: leucine residue
Lys or K: lysine residue
Met or M: methionine residue
Phe or F: phenylalanine residue
Pro or P: proline residue
Ser or S: serine residue
Thr or T: threonine residue
Trp or W: tryptophan residue
Tyr or Y: tyrosine residue
Val or V: valine residue
Abu: 2-aminobutyric acid residue (also referred to as α-aminobutyric residue)
Orn: ornithine residue
Cit: citrulline residue

In the present specification, the amino acid sequence of the "peptide" is described such that the amino acid residue of the N-terminal amino acid is positioned on the left side and the amino acid residue of the C-terminal amino acid is positioned on the right side according to a conventional method. In the "peptide", the amino group of the amino acid residue of the N-terminal amino acid is bonded to a hydrogen atom, and the carbonyl group of the amino acid residue of the C-terminal amino acid is bonded to a hydroxy group, unless otherwise specified. The divalent group of the peptide means a group that is bonded via the amino group of the amino acid residue of the N-terminal amino acid and the carbonyl group of the amino acid residue of the C-terminal amino acid.

In the present specification, in a compound, for example, in a compound represented by the formula (2) and (3), the amino group of the amino acid residue of the N-terminal amino acid is also bonded to a hydrogen atom, and the carbonyl group of the amino acid residue of the C-terminal amino acid is also bonded to a hydroxy group, as to a peptide corresponding to its partial structure, unless otherwise specified.

In the present specification, "R¹" represents a hydrogen atom or tumor antigen peptide B and is preferably tumor antigen peptide B. As for a compound of the formula (1) wherein R¹ is a hydrogen atom, its sequence is not completely the same as a partial sequence of WT1 protein. Specifically, the compound of the formula (1) wherein R¹ is a hydrogen atom is the one in which a cysteine residue is added to the N-terminal side of tumor antigen peptide A, and is therefore not a partial peptide consisting of amino acids of 8 to 35 consecutive residues in the amino acid sequence of human WT1 described in SEQ ID NO: 1.

Examples of the compound of the formula (1) wherein R¹ is a hydrogen atom include the following amino acid sequences:
CRMFPNAPYL (SEQ ID NO: 40),
CCMTWNQMNL (SEQ ID NO: 41),
CCYTWNQMNL (SEQ ID NO: 42),
CALLPAVPSL (SEQ ID NO: 43),
CSLGEQQYSV (SEQ ID NO: 44) and
CRVPGVAPTL (SEQ ID NO: 45).

In the present specification, "X^{a}" and "Y^{a}" independently represent a single bond or a divalent group of a peptide consisting of amino acids of 1 to 4 residues. The sum of the number of amino acid residues of X^{a} and the number of amino acid residues of Y^{a} is an integer of 0 to 4. For example, "the sum is an integer of 0" means that X^{a} and Y^{a} are a single bond. Examples of the case that the sum is an integer of 4 include the case that X^{a} and Y^{a} are independently a divalent group of a peptide consisting of amino acids of 2 residues, the case that X^{a} is a divalent group of a peptide consisting of amino acids of 3 residues and Y^{a} is a divalent group of a peptide consisting of an amino acid of 1 residue, and the case that X^{a} is a divalent group of a peptide consisting of amino acids of 4 residues and Y^{a} is a single bond.

The integer of the sum is preferably 0 to 2, more preferably 0 to 1, most preferably 0. Specifically, the case that both of X^{a} and Y^{a} are a single bond is most preferable.

Examples of the case that the integer of the sum is 2 include the case that X^{a} is a divalent group of a peptide consisting of amino acids of 2 residues and Y^{a} is a single bond, the case that X^{a} and Y^{a} are independently a divalent group of a peptide consisting of an amino acid of 1 residue, and the case that X^{a} is a single bond and Y^{a} is a divalent group of a peptide consisting of amino acids of 2 residues.

Examples of the case that the integer of the sum is 1 include the case that X^{a} is a divalent group of a peptide consisting of an amino acid of 1 residue and Y^{a} is a single bond, and the case that X^{a} is a single bond and Y^{a} is a divalent group of a peptide consisting of an amino acid of 1 residue. Among them, the case that X^{a} is a single bond and Y^{a} is an alanine residue, a leucine residue or a methionine residue is preferable.

In the present embodiment, the pharmaceutical composition comprises a particular WT1 killer peptide and/or WT1 helper peptide or a pharmaceutically acceptable salt thereof, i.e., a peptide comprising an amino acid sequence selected from the group consisting of RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7), VLDFAPPGA (SEQ ID NO: 9), CMTWNQMNL (SEQ ID NO: 3), CYTWNQMNL (SEQ ID NO: 4), CNKRYFKLSHLQMHSRK (SEQ ID NO: 11), CNKRYFKLSHLQMHSRKH (SEQ ID NO: 12), CNKRYFKLSHLQMHSRKHTG (SEQ ID NO: 13), WAPVLDFAPPGASAYGSL (SEQ ID NO: 14), CWAPVLDFAPPGASAYGSL (SEQ ID NO: 15) and WAPVLDFAPPGASAYGSLC (SEQ ID NO: 16) or a pharmaceutically acceptable salt thereof. The pharmaceutical composition according to the present embodiment is not inhibited from containing a peptide or a pharmaceutically acceptable salt thereof other than those described above, and the pharmaceutical composition may further contain a peptide other than those described above, for example, another WT1 killer peptide and/or WT1 helper peptide.

In the present specification, the "WT1 peptide" is a peptide comprising a moiety consisting of consecutive amino acids present in the amino acid sequence of human WT1 described in SEQ ID NO: 1.

The WT1 killer peptide means an MHC class I-restricted WT1 peptide.

In the present specification, "MHC class I-restricted" means a property of inducing CTL by binding to an MHC class I molecule which is class I of major histocompatibility complex (MHC). The "MHC class I-restricted WT1 peptide" is a peptide that is presented as a complex by binding to the MHC class I antigen *in vitro* and/or *in vivo,* and means a peptide that induces CTL as a result of the complex being recognized by precursor T cells.

MHC is called human leukocyte antigen (HLA) for humans. HLA corresponding to the MHC class I molecule is classified into subtypes such as HLA-A, B, Cw, F and G. Examples of "MHC class I restriction" preferably include HLA-A restriction, HLA-B restriction and HLA-Cw restriction.

As for each subtype of HLA, polymorphisms (alleles) are known. Examples of the polymorphism of HLA-A include 27 or more such as HLA-A1, HLA-A2 (A0201, A0206, etc.), and HLA-A24, examples of the polymorphism of HLA-B include 59 or more such as HLA-B7, HLA-B40, and HLA-B4403, and examples of the polymorphism of HLA-Cw include 10 or more such as HLA-Cw0301, HLA-Cw0401, and HLA-Cw0602. Among these polymorphisms, HLA-A2 or HLA-A24 is preferred.

The MHC class I-restricted WT1 peptide (WT1 killer peptide) is also called "MHC class I-restricted WT1 epitope". In the present specification, the "MHC class I-restricted WT1 epitope" means a peptide itself that binds to an MHC class I antigen and is presented as a complex. Specifically, the MHC class I-restricted WT1 peptide produces an MHC class I-restricted WT1 epitope through the intracellular degradation of a conjugate by proteasome such as gamma-interferon-inducible lysosomal thiol reductase (GILT, GLT) and/or protease (proteolysis, reductive cleavage of a disulfide bond), and/or trimming into the optimum number of residues by endoplasmic reticulum aminopeptidase 1 (ERAP1, ER-aminopeptidase 1) *in vitro* and/or *in vivo.* In the production, there is a main possible production process in which, first, the C-terminal amino acid of an MHC class I-restricted WT1 epitope arises as a result of degradation by proteasome and/or protease, and next, the N-terminal amino acid of the MHC class I-restricted WT1 epitope arises as a result of trimming by ERAP1. However, the production process may go through a process other than the production process. ERAP1 is now called ERAAP (ER aminopeptidase associated with antigen presentation) and was formerly called A-LAP, PILS-AP or ARTS-1.

Thus, a peptide consisting of amino acids produced by adding an amino acid to the carbonyl group of the C-terminal amino acid of an MHC class I-restricted WT1 epitope is preferable as the MHC class I-restricted WT1 peptide.

The length of the WT1 killer peptide is not particularly limited as long as it functions as a WT1 killer peptide, and, for example, the one consisting of amino acids of 7 to 30 residues, 7 to 15 residues, 8 to 12 residues, 8 to 11 residues, 8 residues, or 9 residues, or a conjugate thereof is acceptable. The WT1 killer peptide may consist of amino acids of 7 residues or more or 8 residues or more or a conjugate thereof and may consist of amino acids of 30 residues or less, 25 residues or less, 22 residues or less, 20 residues or less, 18 residues or less, 15 residues or less, 12 residues or less, 11 residues or less, 10 residues or less or 9 residues or less or a conjugate thereof.

Examples of the WT1 killer peptide include peptides comprising the amino acid sequences described in RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7), VLDFAPPGA (SEQ ID NO: 9), CMTWNQMNL (SEQ ID NO: 3) and CYTWNQMNL (SEQ ID NO: 4), and peptides that comprise an altered amino acid sequence containing an amino acid residue variation in any amino acid sequence selected from among SEQ ID NOs: 1 to 9 and have CTL inducing activity. The pharmaceutical composition according to the present embodiment may comprise, for example, a peptide comprising an amino acid sequence selected from the group consisting of RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7), VLDFAPPGA (SEQ ID NO: 9), CMTWNQMNL (SEQ ID NO: 3) and CYTWNQMNL (SEQ ID NO: 4) or a pharmaceutically acceptable salt thereof. Also, the pharmaceutical composition according to the present embodiment may comprise, for example, a peptide comprising an amino acid sequence selected from the group consisting of RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7), and VLDFAPPGA (SEQ ID NO: 9) corresponding to HLA subtype A2 type (A-0201, A0206, etc.), or a pharmaceutically acceptable salt thereof, or may comprise, for example, a peptide comprising an amino acid sequence selected from the group consisting of CMTWNQMNL (SEQ ID NO: 3) and CYTWNQMNL (SEQ ID NO: 4) corresponding to HLA subtype A24 type (A-2402, etc.), or a pharmaceutically acceptable salt thereof. Furthermore, the pharmaceutical composition according to the present embodiment may comprise, for example, a peptide consisting of the amino acid sequence of RMFPNAPYL (SEQ ID NO: 2) or a pharmaceutically acceptable salt thereof, may comprise a peptide consisting of the amino acid sequence of YMFPNAPYL (SEQ ID NO: 8) or a pharmaceutically acceptable salt thereof, may comprise a peptide consisting of the amino acid sequence of C-CYTWNQMNL (the bond between C and C represents a disulfide bond, SEQ ID NO: 10) or a pharmaceutically acceptable salt thereof, or may comprise a peptide consisting of the amino acid sequence of CCMTWNQMNL (the bond between C and C represents a disulfide bond, SEQ ID NO: 21) or a pharmaceutically acceptable salt thereof.

In the present specification, the "peptide comprising an amino acid sequence" encompasses a peptide consisting of the amino acid sequence and a peptide in which a further amino acid is added to the N-terminal amino acid and/or C-terminal amino acid of the amino acid sequence. In the case that the "MHC class I-restricted WT1 peptide" is added, a peptide having the addition on the C-terminal side is preferable. In the case that the "MHC class I-restricted WT1 epitope" is added, addition to the C-terminal side is preferable.

The "peptide that comprises an altered amino acid sequence containing an amino acid residue variation in an amino acid sequence and has CTL inducing activity" is also called "altered killer peptide". The altered killer peptide means a peptide that consists of an amino acid sequence in which 1 to 3 amino acids are deleted, substituted and/or added in the amino acid sequence, and induces CTL by binding to MHC class I. Examples of the substitution position of the amino acid to be substituted include, in the case of a peptide consisting of amino acids of 9 residues, position 1 (N terminus), position 2, position 3 and position 9. The number of amino acids to be added (also including inserted) is preferably 1 or 2, more preferably 1. A preferable addition position is the C terminus. The number of amino acids to be deleted is preferably 1. For the variation, the amino acid to be added or the amino acid to be substituted may be a non-natural amino acid other than 20 types of amino acids encoded by a gene.

It is known that the regularity (binding motif) of the amino acid sequence of a peptide that can bind to an HLA antigen exists for each polymorphism of the subtype of HLA. For example, as for the binding motif of HLA-A24, it is known that in a peptide consisting of amino acids of 8 to 11 residues, the amino acid at position 2 is Tyr, Phe, Met or Trp and the amino acid at the C terminus is Phe, Leu, Ile, Trp or Met (J. Immunol., 152, p. 3913, 1994; J. Immunol., 155, p. 4307, 1994; and Immunogenetics, 41, p. 178, 1995). Accordingly, for example, in the case of a peptide consisting of amino acids of 9 residues, it is possible that position 2 is substituted by Tyr, Phe, Met or Trp and/or position 9 is substituted by Phe, Leu, Ile, Trp or Met, and a peptide that has undergone the substitution is preferable as an altered killer peptide. Likewise, as for the binding motif of HLA-A*02:01, it is known that in a peptide consisting of amino acids of 8 to 11 residues, the amino acid at position 2 is Leu or Met and the amino acid at the C terminus is Val or Leu. Therefore, for example, in the case of a peptide consisting of amino acids of 9 residues, it is possible that position 2 is substituted by Leu or Met and/or position 9 is substituted by Val or Leu, and a peptide that has undergone the substitution is preferable as an altered killer peptide.

Examples of the altered killer peptide include the following peptides:
RYFPNAPYL (SEQ ID NO: 22) (see International Publication No. WO 03/106682);
FMFPNAPYL (SEQ ID NO: 23),
RLFPNAPYL (SEQ ID NO: 24),
RMMPNAPYL (SEQ ID NO: 25),
RMFPNAPYV (SEQ ID NO: 26) and
YMFPNAPYL (SEQ ID NO: 7) (see International Publication No. WO 2009/072610)
which are altered killer peptides of RMFPNAPYL (SEQ ID NO: 2);
CYTWNQMNL (SEQ ID NO: 4) (see International Publication No. WO 02/79253)
which is an altered killer peptide of CMTWNQMNL (SEQ ID NO: 3);
Xaa-Met-Thr-Trp-Asn-Gln-Met-Asn-Leu (SEQ ID NO: 27)
(in this sequence, Xaa represents Ser or Ala) and
Xaa-Tyr-Thr-Trp-Asn-Gln-Met-Asn-Leu (SEQ ID NO: 28)
(in this sequence, Xaa represents Ser, Ala, Abu, Arg, Lys, Orn, Cit, Leu, Phe or Asn) (see International Publication No. WO 2004/026897);
AYLPAVPSL (SEQ ID NO: 29) (see International Publication No. WO 2003/106682)
which is an altered killer peptide of ALLPAVPSL (SEQ ID NO: 5);
FLGEQQYSV (SEQ ID NO: 30),
SMGEQQYSV (SEQ ID NO: 31) and
SLMEQQYSV (SEQ ID NO: 32) (see International Publication No. WO 2009/072610)
which are altered killer peptides of SLGEQQYSV (SEQ ID NO: 6); and
RYPGVAPTL (SEQ ID NO: 33) (see International Publication No. WO 2003/106682)
which is an altered killer peptide of RVPGVAPTL (SEQ ID NO: 7).

From the viewpoint of widely covering HLA subtypes, it is preferable that the pharmaceutical composition according to the present embodiment should comprise a plurality of peptides corresponding to different HLA subtypes, respectively. For example, it is preferable to comprise both of the peptide corresponding to HLA subtype A2 type or a pharmaceutically acceptable salt thereof and the peptide corresponding to HLA subtype A24 type or a pharmaceutically acceptable salt thereof. In such a case, for example, the pharmaceutical composition may comprise a compound represented by the formula (I): 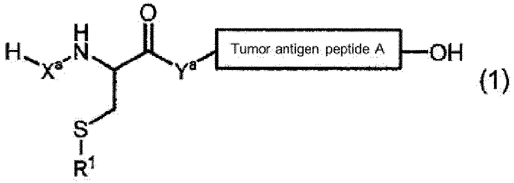 wherein X^{a} and Y^{a} represent a single bond, tumor antigen peptide A represents a peptide consisting of any amino acid sequence selected from among the following amino acid sequences:
RMFPNAPYL (SEQ ID NO: 2), ALLPAVPSL (SEQ ID NO: 5), SLGEQQYSV (SEQ ID NO: 6), RVPGVAPTL (SEQ ID NO: 7) YMFPNAPYL (SEQ ID NO: 8) and VLDFAPPGA (SEQ ID NO: 9),
the amino group of the N-terminal amino acid of the tumor antigen peptide A is bonded to Y^{a} in the formula (1), the carbonyl group of the C-terminal amino acid of the tumor antigen peptide A is bonded to the hydroxy group in the formula (1),
R¹ represents a hydrogen atom or tumor antigen peptide B,
the tumor antigen peptide B differs in sequence from the tumor antigen peptide A and represents a peptide consisting of any amino acid sequence selected from among the following amino acid sequences:
   CMTWNQMNL (SEQ ID NO: 3) and CYTWNQMNL (SEQ ID NO: 4), and the thioether group of the cysteine residue of the tumor antigen peptide B is bonded to the thioether group in the formula (1),
   or a pharmaceutically acceptable salt thereof.

The compound represented by the above formula (I) is excellent in stability against an oxidizing agent or the like in a solution and has given quality as a raw material for medicaments, because the cysteine residue forms a disulfide bond, for example.

In the case that the pharmaceutical composition according to the present embodiment comprises a compound represented by the above formula (I) (conjugate of a WT1 killer peptide) (except for the case that R¹ is a hydrogen atom), the conjugate is degraded by the reductive cleavage of the disulfide bond between the N-terminal cysteine residues by ERAP1 in the body to produce two types of epitopes corresponding to different HLA subtypes. As in the conjugate represented by the formula (I), a conjugate from which a plurality of types of epitopes corresponding to different HLA subtypes are produced is capable of widely coping with different HLA subtypes among subjects and can cover a large population by one conjugate and therefore, can efficiently induce CTL in subjects (see International Publication No. WO 2014/157692).

The "tumor antigen peptide A" according to the present embodiment is an MHC class I-restricted WT1 peptide consisting of amino acids of 7 to 30 residues. In the formula (1), the amino group of the N-terminal amino acid of the tumor antigen peptide A is bonded to Y^{a} in the formula (1), and the carbonyl group of the C-terminal amino acid of the tumor antigen peptide A is bonded to the hydroxy group in the formula (1).

The compound represented by the above formula (1) may be a compound represented by the formula (2): 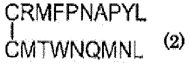
wherein the bond between C and C represents a disulfide bond, or
may be a compound represented by the formula (3): 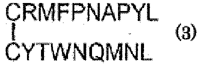
wherein the bond between C and C represents a disulfide bond.

The compound represented by the above formula (1) may be a compound represented by the formula (2): 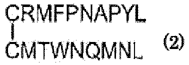
wherein the bond between C and C represents a disulfide bond,
or a pharmaceutically acceptable salt thereof, and
the pharmaceutical composition may further comprise WAPVLDFAPPGASAYGSL (SEQ ID NO: 14) or a pharmaceutically acceptable salt thereof, or
   the compound represented by the formula (1) may be a compound represented by the formula (3): 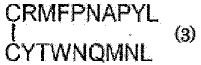
wherein the bond between C and C represents a disulfide bond,
or a pharmaceutically acceptable salt thereof, and
the pharmaceutical composition may further comprise WAPVLDFAPPGASAYGSL (SEQ ID NO: 14) or a pharmaceutically acceptable salt thereof.

The pharmaceutical composition according to the present embodiment may further comprise a WT1 helper peptide. The pharmaceutical composition according to the present embodiment, when comprising a peptide comprising an amino acid sequence selected from the group consisting of CNKRYFKLSHLQMHSRK (SEQ ID NO: 11), CNKRYFKLSHLQMHSRKH (SEQ ID NO: 12), CNKRYFKLSHLQMHSRKHTG (SEQ ID NO: 13), WAPVLDFAPPGASAYGSL (SEQ ID NO: 14), CWAPVLDFAPPGASAYGSL (SEQ ID NO: 15) and WAPVLDFAPPGASAYGSLC (SEQ ID NO: 16), may further comprise a peptide comprising a different amino acid sequence selected from the group and/or an additional WT1 helper peptide other than them.

The WT1 helper peptide means an MHC class II-restricted WT1 peptide.

In the present specification, "MHC class II-restricted" means a property of inducing helper T cells by binding to an MHC class II molecule.

HLA corresponding to the MHC class II molecule is classified into subtypes such as HLA-DR, DQ and DP. Examples of "MHC class II restriction" preferably include HLA-DR restriction, HLA-DQ restriction and HLA-DP restriction.

In the present specification, the "MHC class II-restricted WT1 peptide" means a peptide that induces helper T cells by binding to an MHC class II antigen *in vitro* and/or *in vivo.*

The length of the WT1 helper peptide is not particularly limited as long as it functions as a WT1 helper peptide, and, for example, the one consisting of amino acids of 7 to 30 residues or 14 to 30 residues is acceptable. The WT1 helper peptide may consist of amino acids of 7 residues or more, 8 residues or more, 10 residues or more, 12 residues or more or 14 residues or more and may consist of amino acids of 30 residues or less, 25 residues or less, 22 residues or less or 20 residues or less.

Examples of the WT1 helper peptide include peptides comprising the amino acid sequences described in CNKRYFKLSHLQMHSRK (SEQ ID NO: 11), CNKRYFKLSHLQMHSRKH (SEQ ID NO: 12), CNKRYFKLSHLQMHSRKHTG (SEQ ID NO: 13), WAPVLDFAPPGASAYGSL (SEQ ID NO: 14), CWAPVLDFAPPGASAYGSL (SEQ ID NO: 15), WAPVLDFAPPGASAYGSLC (SEQ ID NO: 16), SGQAYMFPNAPYLPSCLES (SEQ ID NO: 17) (see International Publication No. WO 2007/120673), RSDELVRHHNMHQRNMTKL (SEQ ID NO: 18) (see International Publication No. WO 2007/120673), PGCNKRYFKLSHLQMHSRKHTG (SEQ ID NO: 19) (see International Publication No. WO 2007/120673) and KRYFKLSHLQMHSRKH (SEQ ID NO: 20) (see International Publication No. WO 2005/045027), and peptides that comprise an altered amino acid sequence containing an amino acid residue variation in any amino acid sequence selected from the group consisting of SEQ ID NOs: 11 to 20 and have helper T cell inducing activity. The pharmaceutical composition according to the present embodiment may comprise, for example, a peptide comprising an amino acid sequence selected from the group consisting of CNKRYFKLSHLQMHSRK (SEQ ID NO: 11), CNKRYFKLSHLQMHSRKH (SEQ ID NO: 12), CNKRYFKLSHLQMHSRKHTG (SEQ ID NO: 13), WAPVLDFAPPGASAYGSL (SEQ ID NO: 14), CWAPVLDFAPPGASAYGSL (SEQ ID NO: 15) and WAPVLDFAPPGASAYGSLC (SEQ ID NO: 16) or a pharmaceutically acceptable salt thereof. Also, the pharmaceutical composition according to the present embodiment may comprise, for example, a peptide consisting of the amino acid sequence of CWAPVLDFAPPGASAYGSL (SEQ ID NO: 15) or a pharmaceutically acceptable salt thereof, may comprise a peptide consisting of the amino acid sequence of WAPVLDFAPPGASAYGSLC (SEQ ID NO: 16) or a pharmaceutically acceptable salt thereof, or may comprise a peptide consisting of the amino acid sequence of WAPVLDFAPPGASAYGSL (SEQ ID NO: 14) or a pharmaceutically acceptable salt thereof.

The "peptide comprising an amino acid sequence" means, as mentioned above, a peptide consisting of the amino acid sequence and a peptide in which a further amino acid is added to the N-terminal amino acid and/or C-terminal amino acid of the amino acid sequence. The WT1 helper peptide may contain 1 or 2 or more cysteine residues in the amino acid sequence. A cysteine residue, when added to the amino acid sequence, may be added to the N-terminal side or/and C-terminal side of the amino acid sequence.

In the present specification, the "peptide that comprises an altered amino acid sequence containing an amino acid residue variation in an amino acid sequence and has helper T cell inducing activity" is also called "altered helper peptide". The altered helper peptide means a peptide that consists of an amino acid sequence in which 1 to 3 amino acids are deleted, substituted and/or added in the amino acid sequence, and induces helper T cells by binding to MHC class II. The number of amino acids to be added (also including inserted) is preferably 1 to 3. The number of amino acids to be deleted is preferably 1 to 5. For the variation, the amino acid to be added or the amino acid to be substituted may be a non-natural amino acid other than 20 types of amino acids encoded by a gene.

Examples of the altered helper peptide include the following peptides:
SGQAYMFPNAPYLPSCLES (SEQ ID NO: 35) (see Patent Literature 6),
SGQARMFPNAPYLPSC (SEQ ID NO: 36) and
SGQAYMFPNAPYLPSC (SEQ ID NO: 37)
which are altered helper peptides of SGQARMFPNAPYLPSCLES (SEQ ID NO: 34); and
PGCNKRYFKLSHLQMHSRK (SEQ ID NO: 38),
PGCNKRYFKLSHLQMHSRKH (SEQ ID NO: 39),
CNKRYFKLSHLQMHSRK (SEQ ID NO: 11),
CNKRYFKLSHLQMHSRKH (SEQ ID NO: 12) and
CNKRYFKLSHLQMHSRKHTG (SEQ ID NO: 13)
which are altered helper peptides of PGCNKRYFKLSHLQMHSRKHTG (SEQ ID NO: 19).

The peptide or the compound according to the present embodiment can be produced in accordance with a method described in Examples in the present specification, or a method that is used in usual peptide synthesis. Examples of the production method include methods described in the literatures (Peptide Synthesis, Wiley-Interscience, New York, 1966; The Proteins, Vol 2, Academic Press Inc., New York, 1976; Peptide Synthesis, Maruzen Publishing Co., Ltd., 1975; Basics and Experiments of Peptide Synthesis, Maruzen Publishing Co., Ltd., 1985; and Development of Medicaments, 2, Vol. 14, Peptide Synthesis, Hirokawa-Shoten Ltd., 1991). For example, for a method for producing a compound represented by the formula (1), also see International Publication No. WO 2014/157692.

Examples thereof include a method of performing production in a solid-phase synthesizer by using an Fmoc method or a Boc method, and a method of performing production by sequentially condensing Boc-amino acids or Z-amino acids by a liquid-phase synthesis method (Fmoc represents a 9-fluorenylmethoxycarbonyl group, Boc represents a t-butoxycarbonyl group, and Z represents a benzyloxycarbonyl group).

In an intermediate for producing the peptide or the compound according to the present embodiment, a functional group such as an amino group, a carboxy group, or a mercapto group can be protected with an appropriate protective group and deprotected, if necessary, by using techniques of protection and deprotection. Suitable protective groups, protection methods, and deprotection methods are described in detail in "Protective Groups in Organic Synthesis 2nd Edition (John Wiley & Sons, Inc.; 1990)", etc. Examples of the protective group for the mercapto group include an acetamidomethyl group and a trityl group.

In the case that the peptide or the compound according to the present embodiment has a disulfide bond, the disulfide bond can be formed between two different peptides containing cysteine residues, or between a peptide containing a cysteine residue and cysteine, in accordance with a method that is used in usual peptide chemistry. Examples of the method for forming a disulfide bond include methods described in the literatures (Peptide Synthesis, Wiley-Interscience, New York, 1966; The Proteins, Vol 2, Academic Press Inc., New York, 1976; Peptide Synthesis, Maruzen Publishing Co., Ltd., 1975; Basics and Experiments of Peptide Synthesis, Maruzen Publishing Co., Ltd., 1985; and Development of Medicaments, 2, Vol. 14, Peptide Synthesis, Hirokawa-Shoten Ltd., 1991).

Specifically, in the case that one cysteine residue is contained in a peptide, a compound having a disulfide bond (disulfide compound) can be produced by removing all protective groups including a protective group for a mercapto group on a cysteine side chain, followed by oxidation in an inert solvent. Also, it can be produced by mixing and oxidizing two intermediates having mercapto groups in an appropriate solvent. A known method for forming a disulfide bond in usual peptide synthesis can be appropriately selected as a method for the oxidation. Examples thereof include iodine oxidation, a method of applying air oxidation reaction under alkaline conditions, and a method of forming a disulfide bond by adding an oxidizing agent under alkaline or acidic conditions. Here, examples of the oxidizing agent include iodine, dimethyl sulfoxide (DMSO), and potassium ferricyanide. For example, water, acetic acid, methanol, chloroform, DMF or DMSO, or a mixed solution thereof can be used as the solvent. The oxidation reaction often offers a mixture of symmetric or asymmetric disulfide compounds. The asymmetric disulfide compound of interest can be obtained through purification by various chromatographies or recrystallization. Alternatively, a selective disulfide bond can be formed by mixing an intermediate having an activated mercapto group with an intermediate having a mercapto group. Examples of the intermediate having an activated mercapto group include a mercapto group bonded to a Npys group (3-nitro-2-pyridinesulfenyl group). Alternatively, a selective disulfide bond can be formed by mixing one of the intermediates in advance with, for example, 2,2'-dithiobis(5-nitropyridine), thereby activating the mercapto group, and then adding the other intermediate (Tetrahedron Letters. Vol. 37. No. 9, pp. 1347-1350).

In the case that two or more cysteine residues are contained in a peptide, methods similar to those described above can also be used. In this case, isomers differing in disulfide bond pattern are obtained. A dimer that has formed a disulfide bond between the cysteine residues of interest can be obtained by a particular combination of protective groups on cysteine side chains. Examples of the combination of the protective groups include a MeBzl (methylbenzyl) group and an Acm (acetamidomethyl) group, a Trt (trityl) group and an Acm group, a Npys (3-nitro-2-pyridylthio) group and an Acm group, and a S-Bu-t (S-tert-butyl) group and an Acm group. For example, in the case of the combination of a MeBzl group and an Acm group, examples thereof include a method of first removing the MeBzl group and other protective groups other than those on the cysteine side chains, then subjecting a solution containing a peptide monomer to air oxidation reaction to form a disulfide bond between the deprotected cysteine residues, and subsequently performing deprotection and oxidation with iodine to form a disulfide bond between cysteine residues protected with the Acm group.

The obtained peptide or compound according to the present embodiment can be purified in accordance with a method known to those skilled in the art or a method that is used in usual peptide chemistry. It can be purified by, for example, various chromatographies (e.g., silica gel column chromatography, ion-exchange column chromatography, gel filtration, or reverse-phase chromatography), or recrystallization. For example, an alcohol solvent such as methanol, ethanol or 2-propanol, an ether solvent such as diethyl ether, an ester solvent such as ethyl acetate, an aromatic hydrocarbon solvent such as benzene or toluene, a ketone solvent such as acetone, a hydrocarbon solvent such as hexane, an aprotic solvent such as dimethylformamide or acetonitrile, water, or a mixed solvent thereof can be used as a recrystallization solvent. Methods described in Experimental Chemistry (ed. by The Chemical Society of Japan, Maruzen Publishing Co., Ltd.), Vol. 1, etc. can be used as other purification methods.

A method for purifying a disulfide compound is described in the literatures (Peptide Synthesis, Wiley-Interscience, New York, 1966; The Proteins, Vol 2, Academic Press Inc., New York, 1976; Peptide Synthesis, Maruzen Publishing Co., Ltd., 1975; Basics and Experiments of Peptide Synthesis, Maruzen Publishing Co., Ltd., 1985; and Development of Medicaments, 2, Vol. 14, Peptide Synthesis, Hirokawa-Shoten Ltd., 1991), etc. Among others, HPLC is preferable.

In the case that there are one or more chiral centers in the compound according to the present embodiment, it can be produced by using raw materials (amino acids) having the chiral centers according to a usual method. In order to enhance the optical purity of the compound according to the present embodiment, optical resolution or the like may be performed at an appropriate stage of a production step. The optical resolution method can be performed by, for example, a diastereomer method of allowing the compound according to the present embodiment or its intermediate to form a salt with an optically active acid (e.g., monocarboxylic acid such as mandelic acid, N-benzyloxyalanine, or lactic acid, dicarboxylic acid such as tartaric acid, o-diisopropylidenetartaric acid or malic acid, or sulfonic acid such as camphorsulfonic acid or bromocamphorsulfonic acid) in an inert solvent (e.g., an alcohol solvent such as methanol, ethanol, or 2-propanol, an ether solvent such as diethyl ether, an ester solvent such as ethyl acetate, a hydrocarbon solvent such as toluene, or an aprotic solvent such as acetonitrile, and mixed solvents thereof). In the case that the compound or the intermediate according to the present embodiment has an acidic functional group such as a carboxy group, the optical resolution can also be performed by allowing it to form a salt with optically active amine (e.g., organic amine such as α-phenethylamine, kinin, quinidine, cinchonidine, cinchonine, and strychnine).

A temperature at which the salt is formed is selected from the range from room temperature to the boiling point of the solvent. It is desirable for improving optical purity to temporarily elevate the temperature to near the boiling point of the solvent. In collecting the deposited salt by filtration, yields can be improved, if necessary, by cooling. The amount of the optically active acid or amine used is appropriately in the range of approximately 0.5 to approximately 2.0 equivalents, preferably in the range of around 1 equivalent, with respect to a substrate. An optically active salt with high purity can also be obtained, if necessary, by recrystallizing crystals in an inert solvent (e.g., an alcohol solvent such as methanol, ethanol, or 2-propanol, an ether solvent such as diethyl ether, an ester solvent such as ethyl acetate, a hydrocarbon solvent such as toluene, or an aprotic solvent such as acetonitrile, and mixed solvents thereof). Also, a free form may be obtained, if necessary, by treating an optically resolved salt with an acid or a base by a usual method.

In the present specification, examples of the "pharmaceutically acceptable salt" include acid-addition salts and base-addition salts. Examples of the acid-addition salt include inorganic acid salts such as hydrochloride, hydrobromide, sulfate, hydroiodide, nitrate, and phosphate, and organic acid salts such as citrate, oxalate, acetate, formate, propionate, benzoate, trifluoroacetate, maleate, tartrate, methanesulfonate, benzenesulfonate, and p-toluenesulfonate. Examples of the base-addition salt include inorganic base salts such as sodium salt, potassium salt, calcium salt, magnesium salt, and ammonium salt, and organic base salts such as triethylammonium salt, triethanolammonium salt, pyridinium salt, and diisopropylammonium salt. Further examples thereof include amino acid salts of basic or acidic amino acids such as arginine, aspartic acid, and glutamic acid.

A hydrate and a solvate such as an ethanol solvate, of the peptide or the compound according to the present embodiment or the pharmaceutically acceptable salt thereof are also included in the present embodiment. Further, the pharmaceutical composition according to the present embodiment also encompasses every possible stereoisomer such as every diastereomer and enantiomer, and a crystal form in every form, of the compound represented by the formula (I).

The pharmaceutical composition according to the present embodiment can be used in the treatment or prevention of cancer expressing WT1 gene or cancer accompanied by elevation in the expression level of WT1 gene (WT1-related cancer). Examples of such cancer include leukemia, myelodysplastic syndrome, multiple myeloma, malignant lymphoma, stomach cancer, colorectal cancer, lung cancer, breast cancer, germ cell cancer, liver cancer, skin cancer, urinary bladder cancer, prostate cancer, uterus cancer, uterine cervical cancer, ovary cancer, extragonadal germ cell tumor, brain tumor, brain cancer, extracranial germ cell tumor, bone cancer, pancreatic cancer, head and neck cancer, jaw cancer, esophagus cancer, hypopharynx cancer, larynx cancer, lip and oral cavity cancer, medulloblastoma, melanoma, Merkel cell carcinoma, mesothelioma (such as pleural mesothelioma, pericardial mesothelioma, and peritoneal mesothelioma), sarcoma (such as osteosarcoma, leiomyosarcoma, Kaposi's sarcoma, malignant fibrous histiocytoma, liposarcoma, Ewing's sarcoma, and dermatofibrosarcoma protuberans), neuroblastoma, retinoblastoma, hepatoblastoma, nephroblastoma, glial tumor, cutaneous or intraorbital malignant melanoma, squamous cell cancer, neck squamous cell cancer, intraocular melanoma, biliary tract cancer, colon cancer, duodenum cancer, small intestine cancer, rectal cancer, anus cancer, appendix cancer, bile duct cancer, extrahepatic bile duct cancer, pancreatic islet cell cancer, testis cancer, fallopian tube carcinoma, endometrial carcinoma, uterine cervical carcinoma, vagina carcinoma, vulva carcinoma, Hodgkin's disease, non-Hodgkin's lymphoma, lymphoplasmacytic lymphoma, bronchial adenoma/carcinoid, Burkitt's lymphoma, carcinoid tumor, cerebellar astrocytoma, chronic nasal cavity and nasal sinus cancer, nasopharynx cancer, salivary adenocarcinoma, sublingual adenocarcinoma, parotid adenocarcinoma, endocrine system cancer, thyroid gland cancer, parathyroid gland cancer, adrenal cancer, soft tissue sarcoma, urethra cancer, penis cancer, astrocytoma, basal cell cancer, chronic or acute leukemia including acute myeloid leukemia, chronic myeloid leukemia, acute lymphoblastic leukemia, and chronic lymphoblastic leukemia, pediatric solid cancer, lymphocytic lymphoma, kidney or ureter cancer, renal pelvis carcinoma, central nervous system (CNS) tumor, primary CNS lymphoma, tumor angiogenesis, spinal tumor, brain stem glioma, pituitary adenoma, Kaposi's sarcoma, squamous cell cancer, planocellular cancer, T cell lymphoma, glioblastoma multiforme, malignant melanoma, non-small cell lung cancer, small-cell lung cancer, renal cell cancer, AIDS-related cancer and asbestos-induced cancer. The cancer may be selected from the group listed above, for example, or may be selected from the group consisting of leukemia, myelodysplastic syndrome, multiple myeloma, malignant lymphoma, stomach cancer, colorectal cancer, lung cancer, breast cancer, germ cell cancer, liver cancer, skin cancer, urinary bladder cancer, prostate cancer, uterus cancer, uterine cervical cancer, ovary cancer and brain tumor. The cancer may be selected from the group consisting of, for example, leukemia, myelodysplastic syndrome, multiple myeloma, urinary bladder cancer, brain tumor, breast cancer, lung cancer, colorectal cancer, malignant lymphoma, esophagus cancer, head and neck cancer, liver cancer, ovary cancer, pancreatic cancer, prostate cancer and stomach cancer, may be selected from the group consisting of leukemia, myelodysplastic syndrome and multiple myeloma, or may be selected from the group consisting of myelodysplastic syndrome, breast cancer, lung cancer, colorectal cancer and urinary bladder cancer.

In the present embodiment, the subject may be a human or may be an animal other than a human. It is preferable that the animal other than a human should be a mammal. The subject may be a human having cancer, a human suspected of having cancer, or a human at risk of development of cancer, and it is preferable to be a human having cancer. The subject, when having cancer, is also expressed as a patient.

The peptide or the compound of the present embodiment or the pharmaceutically acceptable salt thereof can be used as an active ingredient for CTL inducing agents in the cellular immunotherapy of cancer, an active ingredient for cancer vaccines or/and an active ingredient for pharmaceutical compositions by adopting a suitable form according to each peptide or compound or each salt.

The pharmaceutical composition, the peptide or the compound of the present embodiment or the pharmaceutically acceptable salt thereof can be administered together with a pharmaceutically acceptable carrier, for example, an appropriate adjuvant, such that the cell-mediated immunity of the peptide or the compound holds true effectively. For similar reasons, the pharmaceutical composition of the present embodiment can comprise a pharmaceutically acceptable carrier, for example, an appropriate adjuvant. Examples of the adjuvant include precipitated adjuvants and oil adjuvants. The precipitated adjuvant refers to an inorganic suspending agent to which a peptide is adsorbed. Examples of the precipitated adjuvant specifically include sodium hydroxide, aluminum hydroxide (Alum), calcium phosphate, aluminum phosphate, aluminum potassium sulfate, HEPES, and carboxyvinyl polymers. The oil adjuvant refers to an oil emulsion that encloses an aqueous solution containing a peptide in mineral oil to form a micelle for emulsification. Examples of the oil adjuvant specifically include liquid paraffin, lanoline, Freund's adjuvants (complete Freund's adjuvant and incomplete Freund's adjuvant), Montanide, and W/O emulsions. Also, for example, the ones described in the literature (Clin. Microbiol. Rev., 7: 277-289, 1994) are applicable as the adjuvant. Specifically, examples thereof include bacterium-derived components, GM-CSF, cytokines such as interleukin-2, interleukin-7 and interleukin-12, plant-derived components, marine organism-derived components, mineral gels such as aluminum hydroxide, lysolecithin, surfactants such as Pluronic polyol, polyanions, peptides, and oil emulsions (emulsion preparations). Examples of the bacterium-derived component include lipid A, its derivative monophosphoryl lipid A, killed bacteria (examples of which include bacteria of the genus *Mycobacterium* such as *Mycobacterium bovis* BCG), bacterium-derived proteins, polynucleotides, Freund's incomplete adjuvant, Freund's complete adjuvant, cell wall skeleton components (e.g., BCG-CWS), and trehalose dimycolate (TDM).

The peptide or the compound of the present embodiment can also be administered as a liposome preparation, a granular preparation bound with beads of several µm in diameter, a preparation bound with a lipid, a W/O emulsion preparation, or the like.

Further, the peptide or the compound (conjugate) of the present embodiment can be administered together with an MHC class II-restricted WT1 peptide (i.e., a helper peptide). Although the conjugate and the helper peptide may be individually administered as a method for the administration together, a cocktail preparation (cocktail agent or cocktail) comprising the conjugate and the helper peptide in one pharmaceutical composition is more preferable. This cocktail preparation comprises a conjugate capable of yielding an MHC class I-restricted WT1 peptide (i.e., a killer peptide) and an MHC class II-restricted WT1 peptide (i.e., a helper peptide). Accordingly, by administering this cocktail preparation containing the helper peptide as a cancer vaccine in cancer immunotherapy, the activation of helper T cells, which are important for enhancement in the functions of other T cells including CTL, is also possible, and the function/drug efficacy (cell-mediated immune competence, etc.) of the conjugate can be improved.

In the method for selecting a potential subject with benefiting from a pharmaceutical composition for treating or preventing cancer (hereinafter, also referred to as the "selection method of the present embodiment") according to claim 1, both the TP53 and the BCOR gene are analysed for mutations. In a further disclosed embodiment, an indication of being a potential subject with benefiting can be provided on the basis of (1) and 1 or 2 or more in combination selected from the group consisting of (2) to (6) given below. By providing an indication of being a potential subject with benefiting from the pharmaceutical composition, it is also possible to predict the response of the subject to the pharmaceutical composition.
(1) A mutation in TP53 gene and BCOR gene
(2) The mRNA expression level of WT1 gene
(3) A karyotype based on revised IPSS (IPSS-R)
(4) The presence or absence of increase in WT1 antigen peptide-specific CD8 T cells
(5) The presence or absence of delayed type hypersensitivity reaction
(6) Change in the ratio of myeloblasts

In the case of providing an indication of being a potential subject with benefiting on the basis of (1) and 1 or 2 or more in combination selected from the group consisting of (2) to (6), the order of implementation is not limited to the order described above. Those skilled in the art can appropriately set the steps to be carried out and an order in consideration of the efficiency of selection, the condition of or burdens on the subject, etc. It is also possible to predict and select an effective combination by artificial intelligence, machine learning, or/and a statistical method, etc. from big data on the treatment of a patient, for example, a disease, a medical state, genome information, a risk factor for treatment and inspection data. For example, the potential subject with benefiting from the pharmaceutical composition may be selected by first carrying out selection based on one or more selected from (1) to (3) which do not require the administration of the pharmaceutical composition or the peptide, narrowing down the potential subject with benefiting from the pharmaceutical composition on the basis of the results, and then carrying out selection based on one or more selected from (4) to (6) which require the administration of the pharmaceutical composition or the peptide. On the other hand, for example, it is also possible to first carry out selection based on one or more selected from (4) to (6), narrow down the potential subject with benefiting from the pharmaceutical composition on the basis of the results, and then carry out selection based on one or more selected from (1) to (3). For example, in the case that the karyotype based on IPSS-R of a subject has already been determined, it is also possible to narrow down the potential subject with benefiting from the pharmaceutical composition by selection based on (3) and then carry out selection based on one or more selected from (1) to (2) and (4) to (6). In the case of carrying out selection that is preferably carried out without the administration of the pharmaceutical composition or the peptide to the same subject after carrying out selection that requires the administration of the pharmaceutical composition or the peptide, it may be performed after a predetermined period passes so that the influence of the administration of the pharmaceutical composition or the peptide is sufficiently reduced.

The sample analyzed in the method of claim 1 is a sample of body fluid such as blood, lymph, ascitic fluid, pleural effusion, sputum, spinal fluid (cerebrospinal fluid), lacrimal fluid, nasal discharge, saliva, urine, vaginal fluid, seminal fluid and joint fluid. The blood includes plasma, serum, and interstitial fluid.

The sample can be collected from the subject on the basis of a method known in the art. For example, blood or lymph can be collected by a known blood collection method. The sample according to the method of claim 1 is a sample of body fluid and may be blood or spinal fluid.

The effect of the pharmaceutical composition for treating or preventing cancer may differ depending on the type of cancer and the condition of a subject, etc., and examples thereof include the prolongation of survival periods, the stabilization of myeloblasts, decrease in cancer cells, the prevention of metastasis and the delay of progression (for myelodysplastic syndrome (MDS) patients, the prolongation of transition periods to acute myeloid leukemia (AML)). The potential subject with benefiting from the pharmaceutical composition includes, for example, a subject whose likelihood of responding to the pharmaceutical composition is high, and a subject whose likelihood of prolonging a survival period is high by the administration of the pharmaceutical composition.

The "WT1 antigen peptide-specific immune response" is immune response that is specifically induced by the administration of a WT1 antigen peptide, etc. The induced "WT1 antigen peptide-specific immune response" can be confirmed by, for example, the positivity of determination results of HLA tetramer assay mentioned later and/or the positivity of delayed hypersensitivity reaction.

### (1) Mutation in TP53 gene and/or BCOR gene

The TP53 gene is a cancer suppressor gene that encodes nuclear protein p53 consisting of 393 amino acids. The BCOR gene is a corepressor of BCL6 and is a gene that specifically inhibits gene expression by binding to a transcriptional factor. It has been reported that both are poor prognostic factors.

In the present specification, the mutation in TP53 gene and/or BCOR gene means mutations in both the TP53 gene and the BCOR gene, a mutation in the TP53 gene, or a mutation in the BCOR gene. The mutation in TP53 gene and/or BCOR gene may be the substitution, deletion or insertion of a nucleotide base or a combination thereof in each nucleotide sequence. The mutation in TP53 gene and/or BCOR gene may be the substitution, deletion or insertion of 1 to 20, 1 to 10, 1 to 8, 1 to 5, 1 to 4, 1 to 3, 1 to 2 or 1 nucleotide base or a combination thereof in each nucleotide sequence.

In the present specification, the TP53 wild type refers to the case that no mutation is present in the TP53 gene, or the case that the TP53 gene, even if a mutation is present, does not lose an original function or is not accompanied by abnormality (including a silent mutation and a synonymous mutation, etc.). The TP53 mutant refers to the case that a mutation is present in the TP53 gene which thus loses an original function or is accompanied by abnormality. The BCOR wild type refers to the case that no mutation is present in the BCOR gene, or the case that the BCOR gene, even if a mutation is present, does not lose an original function or is not accompanied by abnormality (including a silent mutation and a synonymous mutation, etc.). The BCOR mutant refers to the case that a mutation is present in the BCOR gene which thus loses an original function or is accompanied by abnormality. Thus, the TP53 wild type and/or the BCOR wild type includes the case that either TP53 or BCOR is of wild type, and the case that both TP53 and BCOR are of wild type.

The selection method according to claim 1 comprises:
determining the presence or absence of a mutation in TP53 gene and BCOR gene by using a sample collected from the subject; and
providing an indication that the subject is a potential subject with benefiting from the pharmaceutical composition in the case that the subject has TP53 wild type and BCOR wild type.

The subject, the sample, the pharmaceutical composition, etc. are as mentioned above.

Determining the presence or absence of a mutation in TP53 gene and/or BCOR gene is determining "TP53 mutant and/or BCOR mutant" in the case that a difference (mutation) from the wild-type nucleotide sequence is found in a gene corresponding to the TP53 gene and/or the BCOR gene in the subject and the mutation is a mutation that deletes the original function of the wild type or a mutation accompanied by abnormality, and determining "TP53 wild type and BCOR wild type" in the case that no difference (mutation) from the wild-type nucleotide sequence is found or in the case that the mutation is a mutation that causes no change in the transcription level of each gene and the function of a protein encoded by each gene (including a silent mutation and a synonymous mutation, etc.). The difference (mutation) from the wild-type nucleotide sequence may be detected by comparing the nucleotide sequence of the gene corresponding to the TP53 gene and/or the BCOR gene in the subject with the wild-type TP53 gene and/or BCOR gene. The determination of the nucleotide sequence of the gene corresponding to the TP53 gene and/or the BCOR gene in the subject and the comparison with the wild-type nucleotide sequence can be performed by methods known to those skilled in the art. For example, the presence or absence of a mutation can be determined by extracting DNA by a conventional method from a sample, determining the sequence of each gene by next-generation sequencing (NGS) or the like, and comparing it with the corresponding wild-type gene sequence. For example, depending on a mutation, the presence or absence of a mutation can also be determined by PCR-RFLP (restriction fragment length polymorphism) without determining the nucleotide sequence. Also, it may be performed by using, for example, a commercially available DNA mutation/polymorphism detection kit.

The selection method according to claim 1 comprises determining the presence or absence of mutations in the TP53 gene and the BCOR gene, and as a result, comprises providing an indication that the subject is a potential subject with benefiting from the pharmaceutical composition in the case of TP53 wild type and BCOR wild type .

On the other hand, the selection method may comprise, for example, providing, in the case of TP53 mutant and/or BCOR mutant, an indication that the subject is not a potential subject with benefiting from the pharmaceutical composition.

The case of not losing an original function or being not accompanied by abnormality means that each gene is identical or substantially identical to wild type (including a silent mutation and a synonymous mutation, etc.).

Thus, the TP53 gene and the BCOR gene can be used as a marker for providing an indication of whether or not to be a potential subject with benefiting from a pharmaceutical composition for treating or preventing cancer.

### (2) mRNA expression level of WT1 gene

Disclosed is a method for selecting a potential subject with benefiting from a pharmaceutical composition for treating or preventing cancer on the further basis of the mRNA expression level of WT1 gene.

Said method comprises:
determining the mRNA expression level of WT1 gene by using a sample collected from the subject; and
providing an indication that the subject is a potential subject with benefiting from the pharmaceutical composition in the case that the mRNA expression level of WT1 gene is less than a reference value or the reference value or less.

Determining the mRNA expression level of WT1 gene can be carried out by a method known to those skilled in the art, such as quantitative PCR. Also, it may be performed by using, for example, a commercially available kit such as WT1 mRNA Measurement Kit II "Otsuka" (OTSUKA Pharmaceutical Co., Ltd.). For example, RNA is extracted from a sample and subjected to quantitative PCR reaction such as RT-PCR with a real-time PCR apparatus or the like by using primers specific for WT1, and measurement values of WT1 mRNA and housekeeping gene (GAPDH, β-actin, etc.) mRNA as an internal control can be calculated on the basis of a calibration curve. Then, for example, as shown in the expression (expression 1) given below, a WT1 mRNA expression level (copies/µg RNA) can be calculated by multiplying a value obtained by dividing the WT1 mRNA measurement value by the housekeeping gene mRNA measurement value (WT1 mRNA copy number per copy of housekeeping gene mRNA) by an average housekeeping gene mRNA copy number per µg of RNA in healthy adult humans (housekeeping gene mRNA expression level). Thus, the WT1 mRNA expression level (copies/µg RNA) can also be regarded as a value that is calculated according to the following expression (expression 1). $\begin{matrix}WT 1 mRNA expression \\ level \left(copies/μg RNA\right)\end{matrix} = \frac{\begin{matrix}WT 1 mRNA measurement value \\ \left(copies/mL\right)\end{matrix}}{\begin{matrix}Housekeeping gene mRNA measurement value \\ \left(copies/mL\right)\end{matrix}} \times \begin{matrix}Average housekeeping gene mRNA \\ measurement value per μg of RNA \\ in healthy adult humans \\ \left(copies/μg RNA\right)\end{matrix}$

In the case of using GAPDH as a housekeeping gene, for example, RNA is extracted from a sample and subjected to quantitative PCR reaction such as RT-PCR with a real-time PCR apparatus or the like by using primers specific for WT1, and measurement values of WT1 mRNA and GAPDH mRNA can be calculated on the basis of a calibration curve. Then, for example, as shown in the expression given below, a WT1 mRNA expression level (copies/µg RNA) can be calculated by multiplying a value obtained by dividing the WT1 mRNA measurement value by the GAPDH mRNA measurement value (WT1 mRNA copy number per copy of GAPDH mRNA) by an average GAPDH mRNA copy number per µg of RNA in healthy adult humans (GAPDH mRNA expression level). Thus, the WT1 mRNA expression level (copies/µg RNA) can also be regarded as a value that is calculated according to the following expression (expression 2). 2.7 × 10⁷ (copies/µg RNA) is an average GAPDH mRNA measurement value per µg of RNA in healthy adult humans. $\begin{matrix}WT 1 mRNA expression \\ level \left(copies/μg RNA\right)\end{matrix} = \frac{WT 1 mRNA measurement value \left(copies/mL\right)}{GAPDH mRNA measurement value \left(copies/mL\right)} \times 2.7 \times {10}^{7} \left(copies/μg RNA\right)$

The WT1 mRNA expression level (copies/µg RNA) can also be regarded as a value that is calculated from the above formula (expression 2) according to WT1 mRNA Measurement Kit II "Otsuka" (OTSUKA Pharmaceutical Co., Ltd.) and the attached protocol.

In the selection method of the present embodiment, in the case that the mRNA expression level of WT1 gene is less than a reference value or the reference value or less, an indication that the subject is a potential subject with benefiting from the pharmaceutical composition can be provided. The reference value of the mRNA expression level of WT1 gene may be, for example, a value between 50 and 100000 (copies/µg RNA), may be a value between 100 and 50000 (copies/µg RNA), may be a value between 1000 and 20000 (copies/µg RNA), may be a value between 2000 and 10000 (copies/µg RNA), may be a value between 3000 and 10000 (copies/µg RNA), or may be a value between 4000 and 10000 (copies/µg RNA). Also, the reference value of the mRNA expression level of WT1 gene may be, for example, a value of 50 (copies/µg RNA) or more, a value of 100 (copies/µg RNA) or more, a value of 250 (copies/µg RNA) or more, a value of 500 (copies/µg RNA) or more, a value of 750 (copies/µg RNA) or more, a value of 1000 (copies/µg RNA) or more, a value of 1000 (copies/µg RNA) or more, a value of 1250 (copies/µg RNA) or more, a value of 1500 (copies/µg RNA) or more, a value of 1750 (copies/µg RNA) or more, 2000 (copies/µg RNA) or more, a value of 2250 (copies/µg RNA) or more, a value of 2500 (copies/µg RNA) or more, a value of 2750 (copies/µg RNA) or more, a value of 3000 (copies/µg RNA) or more, a value of 3250 (copies/µg RNA) or more, a value of 3500 (copies/µg RNA) or more, a value of 3750 (copies/µg RNA) or more, or a value of 4000 (copies/µg RNA) or more, may be a value of 15000 (copies/µg RNA) or less, a value of 14000 (copies/µg RNA) or less, a value of 13000 (copies/µg RNA) or less, a value of 12000 (copies/µg RNA) or less, a value of 11000 (copies/µg RNA) or less, or a value of 10000 (copies/µg RNA) or less, and may be 50 (copies/µg RNA), 100 (copies/µg RNA), 250 (copies/µg RNA), 500 (copies/µg RNA), 750 (copies/µg RNA), 1000 (copies/µg RNA), 1000 (copies/µg RNA), 1250 (copies/µg RNA), 1500 (copies/µg RNA), 1750 (copies/µg RNA), 2000 (copies/µg RNA), 2250 (copies/µg RNA), 2500 (copies/µg RNA), 2750 (copies/µg RNA), 3000 (copies/µg RNA), 3250 (copies/µg RNA), 3500 (copies/µg RNA), 3750 (copies/µg RNA) or more, 4000 (copies/µg RNA), 15000 (copies/µg RNA), 14000 (copies/µg RNA), 13000 (copies/µg RNA), 12000 (copies/µg RNA), 11000 (copies/µg RNA), or 10000 (copies/µg RNA). In the selection method of the present embodiment, for example, in the case that the mRNA expression level of WT1 gene is less than 4000 (copies/µg RNA) or 4000 (copies/µg RNA) or less, an indication that the subject is a potential subject with benefiting from the pharmaceutical composition may be provided, or in the case that the mRNA expression level of WT1 gene is less than 10000 (copies/µg RNA) or 10000 (copies/µg RNA) or less, an indication that the subject is a potential subject with benefiting from the pharmaceutical composition may be provided. In the selection method of the present embodiment, it is preferable that in the case that the mRNA expression level of WT1 gene is less than a value between 4000 and 10000 (copies/µg RNA) or the value or less, an indication that the subject is a potential subject with benefiting from the pharmaceutical composition should be provided.

In the case that the subject is a myelodysplastic syndrome (MDS) patient, it is preferable that the reference value of the mRNA expression level of WT1 gene should be a value of 100000 (copies/µg RNA) or less, it is more preferable to be a value between 4000 and 10000 (copies/µg RNA), and it is further preferable to be a value of 10000 (copies/µg RNA) or less. In the case that the mRNA expression level of WT1 gene in the sample collected from the subject is less than the reference value or the reference value or less, OS tends to be extended.

Thus, the WT1 gene can be used as a marker for providing an indication of whether or not to be a potential subject with benefiting from a pharmaceutical composition for treating or preventing cancer.

### (3) Karyotype based on revised IPSS (IPSS-R)

Disclosed but not claimed is a method for selecting a potential subject with benefiting from a pharmaceutical composition for treating or preventing cancer on the basis of karyotype based on revised IPSS (IPSS-R).

Said method comprises providing an indication that the subject is a potential subject with benefiting from the pharmaceutical composition in the case that the karyotype based on revised IPSS (IPSS-R, "Greenberg et al., Blood 120, no. 12, p. 2454-2465 (2012)") of the subject is other than being very poor. The selection method may comprise, before this step, determining the karyotype based on IPSS-R of the subject by using a sample collected from the subject.

The karyotype based on IPSS-R can be determined by analysis by a method known to those skilled in the art, such as a G differential staining method or a Q differential staining method. In the case that the karyotype based on IPSS-R of the subject is other than being very poor, an indication that the subject is a potential subject with benefiting from the pharmaceutical composition is provided. The selection method of the present embodiment may provide, for example, an indication that the subject is a potential subject with benefiting from the pharmaceutical composition in the case that the karyotype based on IPSS-R of the subject is good/very good, intermediate or poor, may provide an indication that the subject is a potential subject with benefiting from the pharmaceutical composition in the case of being intermediate or poor, may provide an indication that the subject is a potential subject with benefiting from the pharmaceutical composition in the case of being poor, may provide an indication that the subject is a potential subject with benefiting from the pharmaceutical composition in the case of being good/very good, or may provide an indication that the subject is a potential subject with benefiting from the pharmaceutical composition in the case of being good/very good or intermediate. Alternatively, in the case that the karyotype based on IPSS-R of the subject is very poor, an indication that the subject is not a potential subject with benefiting from the pharmaceutical composition may be provided.

### (4) Presence or absence of increase in WT1 antigen peptide-specific CD8 T cells

Disclosed is a method for selecting a potential subject with benefiting from a pharmaceutical composition for treating or preventing cancer on the further basis of the presence or absence of increase in WT1 antigen peptide-specific CD8 T cells.

Said method comprises:
detecting a WT1 antigen peptide-specific CD8 T cell by using a sample collected from the subject given the pharmaceutical composition or the peptide or the pharmaceutically acceptable salt thereof; and
providing an indication that the subject is a potential subject with benefiting from the pharmaceutical composition in the case that the WT1 antigen peptide-specific CD8 T cell has increased as compared with a sample collected from the subject before administration.
The subject, the sample, the pharmaceutical composition or the peptide or the pharmaceutically acceptable salt thereof, etc. are as mentioned above.

The detection of the WT1 antigen peptide-specific CD8 T cell can be confirmed, for example, by measuring the presence or cell number of the WT1 antigen peptide-specific CD8 T cell by an HLA monomer method, an HLA dimer method, an HLA tetramer method (Int. J. Cancer: 100, 565-570 (2002)), an HLA pentamer method, an HLA dextramer method, ELISPOT, real-time RT-PCR or a limiting dilution method (Nat. Med.: 4, 321-327 (1998)). Thus, detecting a WT1 antigen peptide-specific CD8 T cell may be measuring the presence or cell number of the WT1 antigen peptide-specific CD8 T cell. It is preferable for the detection of the WT1 antigen peptide-specific CD8 T cell to perform measurement by an HLA tetramer method. The HLA tetramer is prepared by biotinylating a complex (HLA monomer) of an HLA α chain and β2 microglobulin associated with a peptide, and binding it to fluorescently labeled avidin for tetramerization. The presence or cell number of the WT1 antigen peptide-specific CD8 T cell can be measured by staining the WT1 antigen peptide-specific CD8 T cell with the HLA tetramer, and analyzing it in a flow cytometer. The HLA monomer method, the HLA dimer method, the HLA pentamer method and the HLA dextramer method can also measure the presence or cell number of the WT1 antigen peptide-specific CD8 T cell on the basis of similar principles.

Detecting a WT1 antigen peptide-specific CD8 T cell may be carried out by reacting a complex of a WT1 peptide and an HLA molecule with the sample, and examining the presence or cell number of a WT1 antigen peptide-specific CD8 T cell recognizing the complex contained in the sample. The complex of a WT1 peptide and an HLA molecule may be selected from the group consisting of, for example, an HLA monomer, an HLA dimer, an HLA tetramer, an HLA pentamer and an HLA dextramer. Here, it is preferable that the HLA molecule should be compatible with HLA of the subject. The HLA molecule may be, for example, an HLA-A24 antigen or an HLA-A2 antigen. Detecting a WT1 antigen peptide-specific CD8 T cell may comprises analysis by a flow cytometry method.

The examination of the presence or cell number of a WT1 antigen peptide-specific CD8 T cell recognizing the complex contained in the sample may be performed, for example, by measuring the ratio of HLA tetramer-bound cells to CD8-positive or CD8/CD3-positive WT1 antigen peptide-specific CD8 T cells.

The presence or cell number of the WT1 antigen peptide-specific CD8 T cell bound with an HLA tetramer can be determined, for example, by measuring the ratio of HLA tetramer-bound cells to CD8-positive cells or CD8/CD3-positive cells.

Here, the CD8-positive cells can be labeled and detected by using, for example, a fluorescently labeled mouse anti-human CD8 monoclonal antibody. Also, the CD3-positive cells can be labeled and detected by using a fluorescently labeled mouse anti-human CD3 monoclonal antibody.

Here, it is necessary for the fluorescent dye used to use the one different from a fluorescent dye used in the HLA tetramer. Specifically, it is necessary to use distinct fluorescent dyes in such a way that in the case of using an HLA tetramer labeled with PE, a mouse anti-human CD8 monoclonal antibody labeled with FITC, and a mouse anti-human CD3 monoclonal antibody labeled with PerCP are used.

Specific operation involves, in the case of measuring the ratio of HLA tetramer-bound cells to CD8-positive cells, for example, contacting a PE-labeled HLA tetramer with a biological sample, then further adding a FITC-labeled mouse anti-human CD8 monoclonal antibody for reaction, and analyzing stained cells in a flow cytometer or a fluorescence microscope. CD8-positive cells (CD8⁺) are selected, and the ratio of tetramer-positive cells (CD8⁺tetramer⁺) among them can be used as the ratio of the WT1 antigen peptide-specific CD8 T cell (following): 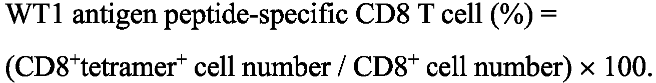 WT1 antigen peptide-specific CD8 T cell (%) = (CD8+tetramer+ cell number / CD8+ cell number) × 100.

In the case of measuring the ratio of HLA tetramer-bound cells to CD3-positive and CD8-positive cells, for example, a PE-labeled HLA tetramer is contacted with a biological sample, then a FITC-labeled mouse anti-human CD8 monoclonal antibody and a PerCP-labeled mouse anti-human CD3 antibody are further added for reaction, and stained cells are analyzed in a flow cytometer or a fluorescence microscope. CD3-positive and CD8-positive cells (CD3⁺CD8⁺) are selected, and the ratio of tetramer-positive cells (CD3⁺CD8⁺tetramer⁺) among them can be used as the ratio of the WT1 antigen peptide-specific CD8 T cell (following): 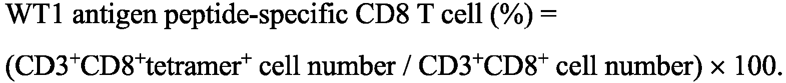 WT1 antigen peptide-specific CD8 T cell (%) = (CD3+CD8+tetramer+ cell number / CD3+CD8+ cell number) × 100.

The selection method of the present embodiment comprises providing an indication that the subject is a potential subject with benefiting from the pharmaceutical composition in the case that the WT1 antigen peptide-specific CD8 T cell has increased as compared with a sample collected from the subject before administration. The sample collected from the subject before administration of the pharmaceutical composition or the peptide or the pharmaceutically acceptable salt thereof is the same type as the sample collected from the subject after administration. For example, in the case that the sample collected from the subject before administration is blood, the sample collected from the subject after administration is also blood. Although the timing of collection of the sample can be appropriately set, for example, a sample collected after most recent administration, immediately to 12 months after administration, immediately to 6 months after administration, immediately to 3 months after administration, immediately to 2 months after administration, immediately to 1 month after administration, immediately to 4 weeks after administration, immediately to 3 weeks after administration, immediately to 2 weeks after administration, immediately to 1 week after administration, immediately to 3 days after administration or immediately to 1 day after administration can be used. For example, it may be immediately after administration or later and/or within 12 months, and, for example, a sample collected less than or within 1 day, 3 days, 1 week, 2 weeks, 3 weeks, 4 weeks, 1 month, 2 months, 3 months or 6 months after administration can be used. For example, a sample collected 1 day, 3 days, 1 week, 2 weeks, 3 weeks, 4 weeks, 1 month, 2 months, 3 months or 6 months or more or more therethan after administration can be used. The number of doses is not particularly limited, and administration may be performed once to 1000 times, once to 100 times, once to 50 times, once to 10 times, once to 5 times, once to 3 times, once to 2 times or once. The number of doses may be, for example, once or more or less than 1000 times, may be less than or within 100 times, 50 times, 10 times, 5 times, 4 times, 3 times or 2 times, or may be 100 times, 50 times, 10 times, 5 times, 4 times, 3 times or 2 times or more or more therethan.

The case that the WT1 antigen peptide-specific CD8 T cell has increased also includes the case that the WT1 antigen peptide-specific CD8 T cell is not detected in the sample collected from the subject before administration and the WT1 antigen peptide-specific CD8 T cell has been detected in the sample collected from the subject after administration.

For example, in the case that the ratio (positive ratio) of the WT1 antigen peptide-specific CD8 T cell among CD8 T cells in the sample collected from the subject after administration has increased by a predetermined ratio or more as compared with the ratio (positive ratio) of the WT1 antigen peptide-specific CD8 T cell among CD8 T cells in the sample collected from the subject before administration, the WT1 antigen peptide-specific CD8 T cell can be regarded as having increased. Also, for example,
(a) in the case that the WT1 antigen peptide-specific CD8 T cell is not detected in the sample collected from the subject before administration, the WT1 antigen peptide-specific CD8 T cell is detected in the sample collected from the subject after administration, and the ratio (positive ratio) thereof is a reference value or more, and/or
(b) in the case that the ratio (positive ratio) of the WT1 antigen peptide-specific CD8 T cell among CD8 T cells in the sample collected from the subject after administration has increased by a predetermined ratio or more as compared with the ratio (positive ratio) of the WT1 antigen peptide-specific CD8 T cell among CD8 T cells in the sample collected from the subject before administration,
the WT1 antigen peptide-specific CD8 T cell can be regarded as having increased.

### Regarding (a)

In the case that the WT1 antigen peptide-specific CD8 T cell is not detected (not present) in the sample collected from the subject before administration, a multiplying factor compared with the cell number of the WT1 antigen peptide-specific CD8 T cell in the sample collected from the subject after administration cannot be calculated. In this case, for example, by being a preset reference value or more, it can be determined that the WT1 antigen peptide-specific CD8 T cell has increased (positive). For example, those skilled in the art can set a range in which the WT1 antigen peptide-specific CD8 T cell is determined as being positive, with reference to results of an already performed test, etc., and appropriately set a value serving as a reference for determining that the WT1 antigen peptide-specific CD8 T cell has increased (positive) as to the number of the WT1 antigen peptide-specific CD8 T cell included in the range. For example, in the case that detecting a WT1 antigen peptide-specific CD8 T cell comprises analysis by a flow cytometry method, a gate including a cell population positive to CD8 and positive to a WT1 peptide is set. Provided that a WT1 antigen peptide-specific CD8 T cell number (event) in the sample collected from the subject after administration included in the range is a reference value or more, for example, 1 or more, 2 or more, 3 or more, 4 or more, 5 or more, 6 or more, 7 or more, 8 or more, 9 or more, 10 or more, 15 or more, or 20 or more, it can be determined that the WT1 antigen peptide-specific CD8 T cell has increased (positive).

### Regarding (b)

In the case that the WT1 antigen peptide-specific CD8 T cell has been detected in the sample collected from the subject before administration, it can be determined that the WT1 antigen peptide-specific CD8 T cell has increased (positive) when the ratio (positive ratio) of the WT1 antigen peptide-specific CD8 T cell among CD8 T cells in the sample collected from the subject after administration has increased by a predetermined ratio or more as compared with the ratio (positive ratio) of the WT1 antigen peptide-specific CD8 T cell among CD8 T cells in the sample collected from the subject before administration. For example, those skilled in the art can set a range in which the WT1 antigen peptide-specific CD8 T cell is determined as being positive, with reference to results of an already performed test, etc., and appropriately set a value serving as a reference for determining that the WT1 antigen peptide-specific CD8 T cell has increased (positive) as to the ratios before and after administration of the number of the WT1 antigen peptide-specific CD8 T cell included in the range. For example, in the case that detecting a WT1 antigen peptide-specific CD8 T cell comprises analysis by a flow cytometry method, a gate including a cell population positive to CD8 and positive to a WT1 tetramer is set. In the case that the ratio (positive ratio) of the WT1 antigen peptide-specific CD8 T cell among CD8 T cells after administration in the sample collected from the subject after administration included in the range is a ratio regarded as being maintained as compared with before administration, or more, it can be determined that the WT1 antigen peptide-specific CD8 T cell has increased (positive). In the case of being the ratio regarded as being maintained, or less, it can be determined that the WT1 antigen peptide-specific CD8 T cell has decreased (negative). The ratio regarded as being maintained can be set to, for example, more than 0.1 to less than 5.0 times, more than 0.5 to less than 2.0 times, more than 0.8 to less than 1.2 times, more than 0.9 to less than 1.1 times or 1.0 times, etc. The ratio regarded as being maintained may be, for example, 0.01, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95 or 1.0 or more or more therethan and may be 5.0 times, 4.5 times, 4.0 times, 3.5 times, 3.0 times, 2.5 times, 2.0 times, 1.8 times, 1.5 times, 1.4 times, 1.3 times, 1.2 times, 1.1 times or 1.0 times or less or less therethan.

In the case of administering the pharmaceutical composition or the peptide or the pharmaceutically acceptable salt thereof a plurality of times, for example, positivity or maintenance may be determined when the WT1 antigen peptide-specific CD8 T cell is determined as having increased (positive) or staying unchanged (maintained) at any point in time after each administration, and negativity may be determined when the WT1 antigen peptide-specific CD8 T cell has decreased (negative) in all points in time.

As mentioned above, it is also possible to evaluate the effect of a candidate substance of a pharmaceutical composition for treating or preventing cancer on the basis of the presence or absence of increase in WT1 antigen peptide-specific CD8 T cells. Specifically, the method for evaluating the effect of a candidate substance of a pharmaceutical composition for treating or preventing cancer according to the present embodiment comprises:
detecting a WT1 antigen peptide-specific CD8 T cell by using a sample collected from a subject given the pharmaceutical composition or a peptide or a pharmaceutically acceptable salt thereof contained in the pharmaceutical composition; and
providing an indication that the candidate substance likely produces an effect on the treatment and prevention of cancer in the case that the WT1 antigen peptide-specific CD8 T cell has increased as compared with a sample collected from the subject before administration.
The subject, the sample, the pharmaceutical composition or the peptide or the pharmaceutically acceptable salt thereof, etc. are as mentioned above.

### (5) Presence or absence of delayed type hypersensitivity reaction

Disclosed is a method for selecting a potential subject with benefiting from a pharmaceutical composition for treating or preventing cancer on the further basis of the presence or absence of delayed type hypersensitivity reaction.

Said method comprises providing an indication that the subject is a potential subject with benefiting from the pharmaceutical composition in the case that delayed type hypersensitivity reaction has been detected in a subject given a plurality of times the pharmaceutical composition or the peptide or the pharmaceutically acceptable salt thereof. The subject, the sample, the pharmaceutical composition or the peptide or the pharmaceutically acceptable salt thereof, etc. are as mentioned above.

When a subject given a pharmaceutical composition or a peptide or a pharmaceutically acceptable salt thereof is given the same pharmaceutical composition or peptide or pharmaceutically acceptable salt thereof, delayed type hypersensitivity reaction may be detected. The delayed type hypersensitivity reaction (DTH reaction) is allergic reaction ascribable to a cell-mediated immunity mechanism that belongs to type IV of Cooms-Gell classification. The presence or absence of this delayed type hypersensitivity reaction can be used as an indication of whether or not the immune response of WT1 antigen peptide-specific CD8 T cells is induced.

The test of delayed type hypersensitivity reaction (DTH test) can be conducted by administrating the same pharmaceutical composition or peptide or pharmaceutically acceptable salt thereof to the subject given once or more the pharmaceutical composition or the peptide or the pharmaceutically acceptable salt thereof. In short, the selection method of the present embodiment administers the same pharmaceutical composition or peptide or pharmaceutically acceptable salt thereof to the subject given once or more the pharmaceutical composition or the peptide or the pharmaceutically acceptable salt thereof, and provides an indication that the subject is a potential subject with benefiting from the pharmaceutical composition in the case that delayed type hypersensitivity reaction has been detected. The former administration once or more is not administration for the DTH test, and the latter administration of the same pharmaceutical composition or peptide or pharmaceutically acceptable salt thereof corresponds to administration for the DTH test. Here, the same pharmaceutical composition or peptide or pharmaceutically acceptable salt thereof can be substantially the same and does not inhibit two or more types of peptides from being separately administered and tested in the DTH test, for example, in the case that the pharmaceutical composition administered once or more is a vaccine in which the two or more types of peptides are mixed. For example, in the case that the pharmaceutical composition administered once or more comprises a peptide consisting of the amino acid sequence of RMFPNAPYL (SEQ ID NO: 2), a peptide consisting of the amino acid sequence of C-CYTWNQMNL (SEQ ID NO: 10), and a peptide consisting of the amino acid sequence of WAPVLDFAPPGASAYGSL (SEQ ID NO: 14), the DTH test may be conducted by separately administering the peptide consisting of the amino acid sequence of RMFPNAPYL (SEQ ID NO: 2) and the peptide consisting of the amino acid sequence of C-CYTWNQMNL (SEQ ID NO: 10) from the peptide consisting of the amino acid sequence of C-CYTWNQMNL (SEQ ID NO: 10). The peptide consisting of the amino acid sequence of RMFPNAPYL (SEQ ID NO: 2) and the peptide consisting of the amino acid sequence of C-CYTWNQMNL (SEQ ID NO: 10) may be in the form of a conjugate as shown in the formula (3).

In the DTH test, the pharmaceutical composition or the peptide or the pharmaceutically acceptable salt thereof is usually administered intradermally. It is preferable that the pharmaceutical composition or the peptide or the pharmaceutically acceptable salt thereof should be administered to a site different from the initial administration site in the subject. The timing of determination after administration for the DTH test can be appropriately set by those skilled in the art, and determination can be performed, for example, 1 hour to 1 week, 12 hours to 5 days, 1 day to 3 days or 2 days after most recent administration. For example, it may be immediately after administration or later and/or within 1 week, may be, for example, less than or within 1 day, 2 days, 3 days, 4 days, 5 days or 6 days after administration, or may be 1 day, 2 days, 3 days, 4 days, 5 days or 6 days or more or more therethan after administration. It is also preferable that the DTH test should be conducted a plurality of times at different timings and the presence or absence of delayed type hypersensitivity reaction should be determined from an average value of the results. The number of times of the DTH test may be, for example, 2 times or more and/or within 20 times, may be, for example, less than or within 2 times, 3 times, 4 times, 5 times, 7 times, 10 times, 15 times or 20 times, or may be, for example, 2 times, 3 times, 4 times, 5 times, 7 times, 10 times or 15 times or more or more therethan. Those skilled in the art can appropriately set the dose of the pharmaceutical composition or the peptide or the pharmaceutically acceptable salt thereof sufficient for detecting delayed type hypersensitivity reaction.

As for the determination of whether or not delayed type hypersensitivity reaction has been detected (presence or absence of delayed type hypersensitivity reaction) in the DTH test, for example, comparing reaction at an administration site of the pharmaceutical composition or the peptide or the pharmaceutically acceptable salt thereof for the DTH test in the subject given once or more the pharmaceutical composition or the peptide or the pharmaceutically acceptable salt thereof is compared with reaction at a non-administration site of the pharmaceutical composition or the peptide or the pharmaceutically acceptable salt thereof for the DTH test in the subject, and in the case that the difference between the reaction at an administration site and the reaction at a non-administration site is a reference value or more, delayed type hypersensitivity reaction can be regarded as having been detected (delayed type hypersensitivity reaction is present). The non-administration site of the pharmaceutical composition or the peptide or the pharmaceutically acceptable salt thereof in the subject may be a site given none of the pharmaceutical composition or the peptide or the pharmaceutically acceptable salt thereof to be administered, or may be a site given only a carrier and a solvent, etc. (vehicle) except for the peptide or the pharmaceutically acceptable salt thereof in the pharmaceutical composition.

The difference in reaction between the administration site and the non-administration site can be drawn from the difference in the major axis of redness between the administration site and the non-administration site (control). Those skilled in the art can appropriately set a reference value for determining that WT1 antigen peptide-specific immune response is positive. For example, in the case that the major axis of redness in the skin at the administration site with respect to the non-administration site is a value between +0.1 mm and 100 mm, or more, delayed type hypersensitivity reaction may be regarded as having been detected. In the case of being a value between +0.5 mm and 50 mm, or more, delayed type hypersensitivity reaction may be regarded as having been detected. In the case of being a value between +1 mm and 10 mm, or more, delayed type hypersensitivity reaction may be regarded as having been detected. In the case of being 2 mm or more, delayed type hypersensitivity reaction may be regarded as having been detected. Additionally, the lower limit value of the range of the major axis of redness by which delayed type hypersensitivity reaction is regarded as having been detected may be 0.1 mm, 0.2 mm, 0.3 mm, 0.4 mm, 0.5 mm, 0.6 mm, 0.7 mm, 0.8 mm, 0.9 mm, or 1.0 mm, and the upper limit value may be 100 mm, 90 mm, 80 mm, 70 mm, 60 mm, 50 mm, 40 mm, 30 mm, 20 mm, 15 mm, or 10 mm. Scores corresponding to the major axis of redness in the skin at the administration site with respect to the non-administration site are preset, and the presence or absence of delayed type hypersensitivity reaction may be determined from the scores. For example, the major axis of redness being less than 2 mm may be set to score 0, being 2 mm or more and less than 5 mm may be set to score +/-, being 5 mm or more and less than 10 mm may be set to score 1, being 10 mm or more and less than 15 mm may be set to score 2, and being 15 mm or more may be set to score 3. For the determination, an average of scores in the subject after administration a plurality of times may be used, or the maximum score in the subject after administration may be used.

As mentioned above, it is also possible to evaluate the effect of a candidate substance of a pharmaceutical composition for treating or preventing cancer on the basis of the presence or absence of delayed type hypersensitivity reaction. Specifically, the method for evaluating the effect of a candidate substance of a pharmaceutical composition for treating or preventing cancer according to the present embodiment comprises providing an indication that the candidate substance of the pharmaceutical composition likely produces an effect on the treatment and prevention of cancer in the case that delayed type hypersensitivity reaction has been detected in a subject given a plurality of times the pharmaceutical composition or a peptide or a pharmaceutically acceptable salt thereof contained in the pharmaceutical composition.

It comprises providing an indication that the candidate substance likely produces an effect on the treatment and prevention of cancer in the case that delayed type hypersensitivity reaction has been detected in a subject given a plurality of times the pharmaceutical composition or a peptide or a pharmaceutically acceptable salt thereof contained in the pharmaceutical composition. The subject, the sample, the pharmaceutical composition or the peptide or the pharmaceutically acceptable salt thereof, etc. are as mentioned above.

### (6) Change in ratio of myeloblasts

The selection method of the present embodiment comprises providing an indication that the subject is a potential subject with benefiting from the pharmaceutical composition in the case that a value obtained by dividing the ratio of myeloblasts in a sample collected from the subject given the pharmaceutical composition or the peptide or the pharmaceutically acceptable salt thereof by the ratio of myeloblasts in a sample collected from the subject before administration of the pharmaceutical composition or the peptide or the pharmaceutically acceptable salt thereof is less than a reference value or the reference value or less. The subject, the sample, the pharmaceutical composition or the peptide or the pharmaceutically acceptable salt thereof, etc. are as mentioned above.

As change in the ratio of myeloblasts in a sample such as bone marrow fluid is smaller between before and after administration of the pharmaceutical composition or the peptide or the pharmaceutically acceptable salt thereof, myeloblasts are regarded as being more stable. For example, in the case that a ratio obtained by dividing the ratio of myeloblasts in the subject after administration of the pharmaceutical composition or the peptide or the pharmaceutically acceptable salt thereof by the ratio of myeloblasts in the subject before administration of the pharmaceutical composition or the peptide or the pharmaceutically acceptable salt thereof (hereinafter, also referred to as the ratio of change in myeloblasts) is 0% to 300% or less, 0% to 200% or less, 0% to 150% or less, 0% to 100% or less, or 0% to 50% or less, an indication that the subject is a potential subject with benefiting from the pharmaceutical composition may be provided. Here, the ratio of change in myeloblasts may be, for example, 0% or more or 300% or less or less therethan, may be, for example, 50%, 100%, 150%, 200% or 250% or more or more therethan, or may be 50%, 100%, 150%, 200% or 250% or less or less therethan.

For example, on the basis of an average ratio of change in myeloblasts at one point in time to several points in time, it may be determined as being less than the reference value or the reference value or less, or on the basis of the maximum value of the ratio of change in myeloblasts at one point in time to several points in time, it may be determined as being less than the reference value or the reference value or less. For example, in the case that a shift is made with less than the reference value or the reference value or less at two points in time or more, it may be determined as being less than the reference value or the reference value or less. The point in time means the timing of measurement of the ratio of myeloblasts after administration of the pharmaceutical composition or the peptide or the pharmaceutically acceptable salt thereof. The timing of measurement of the ratio of myeloblasts may be, for example, every 1 day to 365 days, every 1 day to 180 days, every 1 day to 90 days, every 1 day to 60 days, every 1 day to 30 days, or every 1 day to 4 weeks. The timing of measurement may be, for example, every 1 day or more or less than 365 days, may be every 180 days, 90 days, 60 days, 30 days, 4 weeks, 3 weeks, 2 weeks, 1 weeks, 3 days or 1 day or more or more therethan, or may be 180 days, 90 days, 60 days, 30 days, 4 weeks, 3 weeks, 2 weeks, 1 week or 3 days or less or less therethan. For example, in the case that the ratio of change in myeloblasts up to 100 days after treatment shifts with 150% or less at two points in time or more, the ratio of change in myeloblasts can be determined as being 150% or less.

### (7) Sex difference

The selection method of the present embodiment can provide an indication of being a potential subject with benefiting on the basis of 1 or 2 or more in combination selected from the group consisting of (1) to (6) described above. It may further comprise providing an indication that the subject is a potential subject with benefiting from the pharmaceutical composition in the case that the sex of the subject is male (in the case of a human subject, a male human).

### (Disclosed but not claimed method for treating or preventing cancer)

The method for treating or preventing cancer of the present embodiment comprises:
selecting a potential subject with benefiting from a pharmaceutical composition for treating or preventing cancer by the selection method mentioned above; and
administering the pharmaceutical composition to the selected subject.

The method for treating or preventing cancer of the present embodiment comprises: determining a subject to which a pharmaceutical composition for treating or preventing cancer is administered by the selection method based on 1 or 2 or more in combination selected from the group consisting of (1) to (6) mentioned above; and administering the pharmaceutical composition. Also, the method for treating or preventing cancer of the present embodiment comprises: determining whether or not to administer a pharmaceutical composition for treating or preventing cancer to a subject is administered by the selection method based on 1 or 2 or more in combination selected from the group consisting of (1) to (6) mentioned above; and administering the pharmaceutical composition to the subject. Thus, the method for treating or preventing cancer of the present embodiment comprises: for example, as mentioned above in (1), determining the presence or absence of a mutation in TP53 gene and/or BCOR gene; and administering the pharmaceutical composition for treating or preventing cancer to a subject having TP53 wild type and/or BCOR wild type. The method for treating or preventing cancer of the present embodiment comprises: for example, as mentioned above in (2), determining the mRNA expression level of WT1 gene; and administering the pharmaceutical composition for treating or preventing cancer to a subject whose mRNA expression level of WT1 gene is less than a reference value or the reference value or less. The method for treating or preventing cancer of the present embodiment comprises, for example, as mentioned above in (3), administering the pharmaceutical composition for treating or preventing cancer to a subject whose karyotype based on IPSS-R is other than being very poor. The method for treating or preventing cancer of the present embodiment comprises: for example, as mentioned above in (4), detecting a WT1 antigen peptide-specific CD8 T cell by using a sample collected from a subject given the pharmaceutical composition or the peptide or the pharmaceutically acceptable salt thereof; and administering the pharmaceutical composition to a subject in which the WT1 antigen peptide-specific CD8 T cell has increased as compared with a sample collected from the subject before administration. The method for treating or preventing cancer of the present embodiment comprises, for example, as mentioned above in (5), administering the pharmaceutical composition to a subject in which delayed type hypersensitivity reaction has been detected by administering the pharmaceutical composition or the peptide or the pharmaceutically acceptable salt thereof a plurality of times. The method for treating or preventing cancer of the present embodiment comprises, for example, as mentioned above in (6), administering the pharmaceutical composition to a subject in which a value obtained by dividing the ratio of myeloblasts after administration of the pharmaceutical composition or the peptide or the pharmaceutically acceptable salt thereof by the ratio of myeloblasts before administration of the pharmaceutical composition or the peptide or the pharmaceutically acceptable salt thereof is less than a reference value or the reference value or less.

In the method for treating or preventing cancer of the present embodiment, the dose of the pharmaceutical composition of the present embodiment in a preparation can be appropriately adjusted depending on a disease to be treated, the age and body weight of a patient, etc., but may be 0.0001 mg to 1000 mg, may be 0.001 mg to 1000 mg, or may be 0.1 mg to 10 mg. For example, one dose may be 1.75 mg to 17.5 mg, 3.5 mg to 10.5 mg or 10.5 mg. Also, one dose may be 0.0001 mg or more, 0.0005 mg or more, 0.001 mg or more, 0.005 mg or more, 0.01 mg or more, 0.05 mg or more, 0.1 mg or more, 0.25 mg or more, 0.5 mg or more, 0.75 mg or more, 1.0 mg or more, 1.25 mg or more, 1.5 mg or more, 1.75 mg or more, 2.0 mg or more, 2.25 mg or more, 2.5 mg or more, 2.75 mg or more, 3.0 mg or more, 3.25 mg or more, 3.5 mg or more, 3.75 mg or more, 4.0 mg or more, 4.25 mg or more, 5.5 mg or more, 5.75 mg or more, or 6.0 mg or more and may be 1000 mg or less, 750 mg or less, 500 mg or less, 250 mg or less, 125 mg or less, 100 mg or less, 50 mg or less, mg or less, 40 mg or less, 35 mg or less, 30 mg or less, 25 mg or less, 20 mg or less, 15 mg or less, or 10 mg or less.

Examples of an administration method include intradermal administration, subcutaneous administration, intramuscular administration, intravenous administration, and transdermal administration. Intradermal administration and subcutaneous administration which efficiently induce CTL are preferable. The number of doses and dosing intervals can be appropriately adjusted depending on a disease to be treated or prevented and the difference among individual patients, but are usually a plurality of times, and it is preferable to perform administration once a few days to a few months. For example, administration may be performed once every 1 day to 6 months, may be performed once every 3 days to 3 months, may be performed once every 1 week to 4 weeks, may be performed once 2 weeks to 4 weeks, or may be performed once every 6 weeks, 5 weeks, 4 weeks, 3 weeks, 2 weeks or 1 week. Also, administration may be performed every 1 day, 2 days, 3 days, 4 days, 5 days, 6 days, 1 week, 2 weeks, 3 weeks, 4 weeks, 5 weeks, 6 weeks, 7 weeks, 1 month, 2 months, 3 months, 4 months, 5 months, or 6 months at the minimum and once every 12 months, 10 months, 8 months, 6 months, 5 months, 4 months, 3 months, 2 months, 1 month, 6 weeks, 5 weeks, 4 weeks, 3 weeks, 2 weeks, 1 week, 6 days or 5 days at the maximum.

The timing of the administration may be changed after a lapse of a predetermined period, for example, after a lapse of 1 month, 2 months, 3 months, 4 months, 5 months, 6 months, 7 months, 8 months, 9 months, 10 months, 11 months or 12 months. For example, administration may be performed once every 2 weeks for the first 6 months, performed once every 2 weeks from 1 month or later to 5 months, and performed once every 2 weeks to 4 weeks on or after 6 months. Administration may be performed every 2 weeks for the first 6 months and then performed every 2 weeks to 4 weeks.

The present disclosed but not claimed embodiment also includes a pharmaceutical composition for use in a method for treating or preventing cancer. The pharmaceutical composition, the method for treating or preventing cancer, etc. are as mentioned above.

In the present disclosed but not claimed embodiment, a method for determining a subject to which a pharmaceutical composition for treating or preventing cancer is administered is also included. The subject can be determined on the basis of 1 or 2 or more in combination selected from the group consisting of (1) to (6) mentioned above. The method comprises: for example, as mentioned above in (1), determining the presence or absence of a mutation in TP53 gene and/or BCOR gene; and determining a subject having TP53 wild type and/or BCOR wild type as the subject to which a pharmaceutical composition for treating or preventing cancer is administered. The method comprises: for example, as mentioned above in (2), determining the mRNA expression level of WT1 gene; and determining a subject whose mRNA expression level of WT1 gene is less than a reference value or the reference value or less as the subject to which a pharmaceutical composition for treating or preventing cancer is administered. The method comprises, for example, as mentioned above in (3), determining a subject whose karyotype based on IPSS-R is other than being very poor as the subject to which a pharmaceutical composition for treating or preventing cancer is administered. The method comprises: for example, as mentioned above in (4), detecting a WT1 antigen peptide-specific CD8 T cell by using a sample collected from a subject given the pharmaceutical composition or the peptide or the pharmaceutically acceptable salt thereof; and determining a subject in which the WT1 antigen peptide-specific CD8 T cell has increased as compared with a sample collected from the subject before administration as the subject to which a pharmaceutical composition for treating or preventing cancer is administered. The method comprises, for example, as mentioned above in (5), determining a subject in which delayed type hypersensitivity reaction has been detected by administering the pharmaceutical composition or the peptide or the pharmaceutically acceptable salt thereof a plurality of times as the subject to which a pharmaceutical composition for treating or preventing cancer is administered. The method comprises, for example, as mentioned above in (6), determining a subject in which a value obtained by dividing the ratio of myeloblasts after administration of the pharmaceutical composition or the peptide or the pharmaceutically acceptable salt thereof by the ratio of myeloblasts before administration of the pharmaceutical composition or the peptide or the pharmaceutically acceptable salt thereof is less than a reference value or the reference value or less as the subject to which a pharmaceutical composition for treating or preventing cancer is administered.

In the present disclosed but not claimed embodiment, a method for determining whether or not to administer a pharmaceutical composition for treating or preventing cancer is also included. Whether or not to administer a pharmaceutical composition for treating or preventing cancer can be determined on the basis of 1 or 2 or more in combination selected from the group consisting of (1) to (6) mentioned above. The method comprises: for example, as mentioned above in (1), determining the presence or absence of a mutation in TP53 gene and/or BCOR gene by using a sample collected from a subject; and determining that in the case of TP53 wild type and/or BCOR wild type, the pharmaceutical composition for treating or preventing cancer is administered to the subject and in the case of TP53 mutant and/or BCOR mutant, the pharmaceutical composition for treating or preventing cancer is not administered to the subject. The method comprises: for example, as mentioned above in (2), determining the mRNA expression level of WT1 gene in a sample collected from a subject; and determining that in the case that the mRNA expression level of WT1 gene is less than a reference value or the reference value or less, the pharmaceutical composition for treating or preventing cancer is administered to the subject and in the case that the mRNA expression level of WT1 gene is reference value or more or more than the reference value, the pharmaceutical composition for treating or preventing cancer is not administered to the subject. The method comprises, for example, as mentioned above in (3), determining that in the case that the karyotype based on IPSS-R of a subject is other than being very poor, the pharmaceutical composition for treating or preventing cancer is administered to the subject and in the case that the karyotype based on IPSS-R of the subject is very poor, the pharmaceutical composition for treating or preventing cancer is not administered to the subject. The method comprises: for example, as mentioned above in (4), detecting a WT1 antigen peptide-specific CD8 T cell by using a sample collected from a subject given the pharmaceutical composition or the peptide or the pharmaceutically acceptable salt thereof; and determining that in the case that the WT1 antigen peptide-specific CD8 T cell has increased as compared with a sample collected from the subject before administration, the pharmaceutical composition is administered to the subject and in the case that the WT1 antigen peptide-specific CD8 T cell has been maintained or has decreased, the pharmaceutical composition is not administered to the subject. The method comprises, for example, as mentioned above in (5), determining that in the case that delayed type hypersensitivity reaction has been detected in a subject by administering the pharmaceutical composition or the peptide or the pharmaceutically acceptable salt thereof a plurality of times, the pharmaceutical composition is administered to the subject and in the case that delayed type hypersensitivity reaction is not detected in a subject, the pharmaceutical composition is not administered to the subject. The method comprises, for example, as mentioned above in (6), determining that in the case that a value obtained by dividing the ratio of myeloblasts after administration of the pharmaceutical composition or the peptide or the pharmaceutically acceptable salt thereof by the ratio of myeloblasts before administration of the pharmaceutical composition or the peptide or the pharmaceutically acceptable salt thereof is less than a reference value or the reference value or less, the pharmaceutical composition is administered to the subject and in the case of being a reference value or more or more than the reference value, the pharmaceutical composition is not administered to the subject.

In the present disclosed but not claimed embodiment, a method for screening for a subject to which a pharmaceutical composition for treating or preventing cancer should be administered is also included. The screening can be carried out on the basis of 1 or 2 or more in combination selected from the group consisting of (1) to (6) mentioned above. The method comprises: for example, as mentioned above in (1), determining the presence or absence of a mutation in TP53 gene and/or BCOR gene; and selecting a subject having TP53 wild type and/or BCOR wild type. The method comprises: for example, as mentioned above in (2), determining the mRNA expression level of WT1 gene; and selecting a subject whose mRNA expression level of WT1 gene is less than a reference value or the reference value or less. The method comprises, for example, as mentioned above in (3), selecting a subject whose karyotype based on IPSS-R is other than being very poor. The method comprises: for example, as mentioned above in (4), detecting a WT1 antigen peptide-specific CD8 T cell by using a sample collected from a subject given the pharmaceutical composition or the peptide or the pharmaceutically acceptable salt thereof; and selecting a subject in which the WT1 antigen peptide-specific CD8 T cell has increased as compared with a sample collected from the subject before administration. The method comprises, for example, as mentioned above in (5), selecting a subject in which delayed type hypersensitivity reaction has been detected by administering the pharmaceutical composition or the peptide or the pharmaceutically acceptable salt thereof a plurality of times. The method comprises, for example, as mentioned above in (6), selecting a subject in which a value obtained by dividing the ratio of myeloblasts after administration of the pharmaceutical composition or the peptide or the pharmaceutically acceptable salt thereof by the ratio of myeloblasts before administration of the pharmaceutical composition or the peptide or the pharmaceutically acceptable salt thereof is less than a reference value or the reference value or less.

### Examples

Hereinafter, the present invention will be described in detail with reference to Examples. However, the present invention is not limited by these by any means.

### [Example 1: Subject in this Example and effect of WT1 peptide cocktail vaccine]

In the following Example, among recurrent/refractory myelodysplastic syndrome (MDS) patients from which informed consent was obtained, the number of patients who participated in the phase 1 and phase 2 clinical trials of a cocktail vaccine in a W/O emulsion form containing the WT1 killer peptide conjugate shown in the following formula (3) and the WT1 helper peptide represented by WAPVLDFAPPGASAYGSL (SEQ ID NO: 14) (see International Publication No. WO 2014/157692; hereinafter, also referred to as a WT1 peptide cocktail vaccine) was 47 cases, and the breakdown regarding HLA type thereof was 12 cases of HLA-A*02:01- or HLA-A*02:06-positive myelodysplastic syndrome (MDS) patients, 28 cases of HLA-A*24:02-positive MDS patients, 5 cases of HLA-A*02:01-positive and HLA-A*24:02-positive MDS patients, and 2 cases of HLA-A*02:06-positive and HLA-A*24:02-positive MDS patients, as a rule, unless otherwise specified. The cases in the phase 1 trial included 7 cases of high-risk patients (H) as well as 5 cases of low-risk patients (L), and the cases in the phase 2 trial were 35 cases of high-risk patients (H). In this Example, only the high-risk patients were used as subjects, and the trials were conducted by using 42 cases of azacytidine unresponsive high-risk patients (total of 7 cases from the phase 1 trial and 35 cases from the phase 2 trial) as subjects. The high-risk patients correspond to intermediate to very high patients in the risk classification of revised IPSS (IPSS-R). In the 42 cases of azacytidine unresponsive high-risk patients, 40 cases of patients who did not respond to or no longer responded to azacytidine, and 2 cases that were not able to continue administration due to the adverse reactions of azacytidine were included. 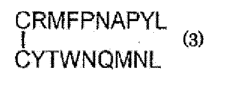 wherein the bond between C and C represents a disulfide bond.

In the phase 1 and phase 2 clinical trials, the WT1 peptide cocktail vaccine was intradermally administered (ID) every 2 weeks for 6 months as vaccine induction. Then, administration every 2 weeks to 4 weeks was continued until the administration was discontinued. The phase 1 trial was conducted at a dose of 3.5 mg (2 mg of the WT1 killer peptide conjugate + 1.5 mg of the WT1 helper peptide) for cohort 1 and at a dose of 10.5 mg (6 mg of the WT1 killer peptide conjugate + 4.5 mg of the WT1 helper peptide) for cohort 2. The phase 2 trial was conducted at a recommended dose of 10.5 mg (6 mg of the WT1 killer peptide conjugate + 4.5 mg of the WT1 helper peptide).

The median survival time (mOS: median overall survival) in this test was 8.6 months (90% confidence interval: 6.8-11.1 months) and was found to have the tendency to be prolonged as compared with 5.6 months of the historical data (95% confidence interval: 5.0-7.2 months, Prebet et al., Journal of Clinical Oncology 29, no. 24, p. 3322-3327 (2011)).

### [Example 2: Effect of WT1 peptide vaccine and influence of karyotype]

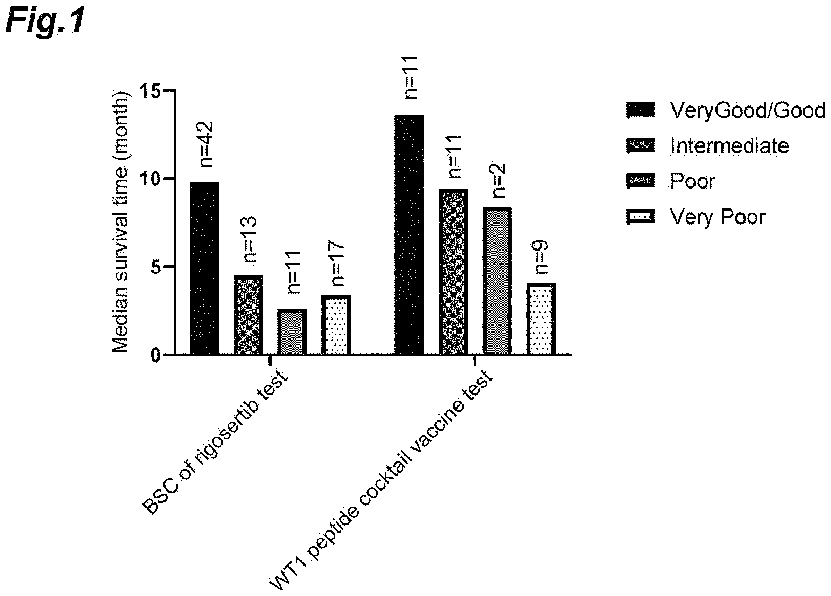

Results of comparing the test results of the WT1 peptide cocktail vaccine in Example 1 with a control (BSC of the rigosertib test) in the ONTIME test of rigosertib (phase 3 clinical trial, Table S1 "MDS cytogenetic prognosis" of Garcia-Manero et al., The Lancet Oncology 17, no. 4, p. 496-508 (2016)) are shown in Figure 1. In consideration of difference from a patient population in the phase 3 trial of rigosertib, the comparison was carried out in 33 cases by excluding 2 unresponsive cases ascribable to the adverse reactions of azacytidine and 6 high-risk cases with gemmule number < 5% or less, and 1 case with unknown karyotype from 42 cases of Example 1.

mOS on karyotype basis tended to be long in the good/very good to poor groups except for karyotype of being very poor, as compared with BSC of the rigosertib test, suggesting the possibility that the prolongation of mOS was brought about by the effect of the WT1 peptide cocktail vaccine. In the group with karyotype of being very poor, mOS of the WT1 peptide cocktail vaccine administration group was equivalent to BSC of the rigosertib test.

### [Example 3: Search for gene biomarker for selecting potential patient with benefiting from treatment with WT1 peptide vaccine]

A gene test associated with myelodysplastic syndrome (MDS) was carried out as to 29 cases from which informed consent was obtained among the 42 high-risk cases described in Example 1. Among the 29 cases, 28 cases except for 1 case which was an unresponsive case ascribable to the adverse reactions of azacytidine was used in the subsequent gene analysis. Bone marrow fluid was collected from the patients within 28 days before the start of administration of the WT1 peptide cocktail vaccine.

### DNA extraction from bone marrow fluid and quality evaluation of specimen

The extraction of DNA was performed from 0.5 mL of a bone marrow fluid sample using Wizard Genomic DNA purification Kit (Promega Corp.) according to the protocol attached to this kit (3A Isolating Genomic DNA from whole blood).

Whether a clear band was able to be detected in a high-molecular region by agarose gel electrophoresis was confirmed as the quality evaluation of the extracted DNA. Fluorescence intensity was measured in a plate reader using Quant-iT PicoGreen dsDNA Assay Kit (Life Technologies Corp.), and a concentration was calculated from a calibration curve.

### Assay by next-generation sequencing (NGS)

Assay was conducted by next-generation sequencing (NGS) using TruSight Myeloid Sequencing Panel (Illumina, Inc.) targeting mutations associated with myeloid malignancies such as acute myeloid leukemia (AML), myelodysplastic syndrome (MDS), myeloproliferative neoplasm (MPN), chronic myeloid leukemia (CML), chronic myelomonocytic leukemia (CMML), and juvenile myelomonocytic leukemia (JMML). For the genomic DNA extracted from the bone marrow fluid, the sequence targeted by TruSight Myeloid Sequencing Panel was analyzed. A region to be measured was amplified by using the panel to prepare a library. Then, a strand length distribution was confirmed by electrophoresis analysis using Agilent DNA1000 Kit (Agilent Technologies, Inc.), and quantification was performed by using a real-time PCR apparatus (Applied Biosystems). The quantification results were corrected with an average library size, and a library concentration was calculated. Nucleotide sequences were determined in MiSeq (Illumina, Inc.). The panel covers 40 genes shown in the following Table 1.

**[Table 1]**

| | | | | |
|---|---|---|---|---|
| CEBPA | SF3B1 | ASXL1 | IDH1 | NOTCH1 |
| SMC1A | ATRX | IDH2 | NPM1 | SMC3 |
| BCOR | DNMT3A | NRAS | SRSF2 | BCORL1 |
| ETV6/TEL | JAK2 | STAG2 | BRAF | EZH2 |
| PHF6 | TET2 | FBXW7 | KDM6A | TP53 |
| CBL | FLT3 | KIT | PTPN11 | U2AF1 |
| CBLB | GATA1 | KRAS | RAD21 | WT1 |
| GATA2 | RUNX1 | ZRSR2 | GNAS | MPL |

### Bioinformatics analysis

The nucleotide sequences determined in the MiSeq (Illumina, Inc.) were analyzed for gene mutations by bioinformatics analysis using MiSeq Reporter (Illumina, Inc.).

### Results

The results of gene mutation analysis are shown in Table 2. In the table, the thick horizontal line is a line that represents mOS, and the survival period (OS: overall survival) gets longer in the order from the upper toward the lower. The description about the IPSS-R karyotype of patients with IPSS-R karyotype of being good/very good or intermediate is omitted.

**[Table 2]**

| ID | IPSS-R karyotype | TP53 | BCOR |
|---|---|---|---|
| 1 | Very Poor | X | |
| 2 | Very Poor | X | |
| 3 | Very Poor | X | X |
| 4 | | | X |
| 5 | Very Poor | X | |
| 6 | Very Poor | X | |
| 7 | | | X |
| 8 | | | |
| 9 | | | |
| 10 | | | X |
| 11 | Very Poor | X | |
| 12 | Very Poor | X | |
| 13 | | | |
| 14 | | | |
| 15 | Poor | X | |
| 16 | | | |
| 17 | Poor | | |
| 18 | | | |
| 19 | | | |
| 20 | | | |
| 21 | | | |
| 22 | | | |
| 23 | Very Poor | | |
| 24 | | | |
| 25 | | | |
| 26 | | | |
| 27 | | | |
| 28 | | | |
| 29 | | | |

It was found for cases having TP53 mutant or BCOR mutant that OS tended to be short. This suggested the possibility that the presence or absence of mutations in TP53 and BCOR gene is useful as gene markers for selecting potential patients with benefiting from treatment with the WT1 peptide vaccine. Also, a tendency to be many cases with karyotype of being very poor was seen in a population having short OS.

### [Example 4: Usefulness of TP53/BCOR gene mutation as gene marker]

In order to verify the usefulness of the presence or absence of mutations in TP53 and BCOR gene as gene markers, the comparison of survival curves based on the presence or absence of the mutations was performed.

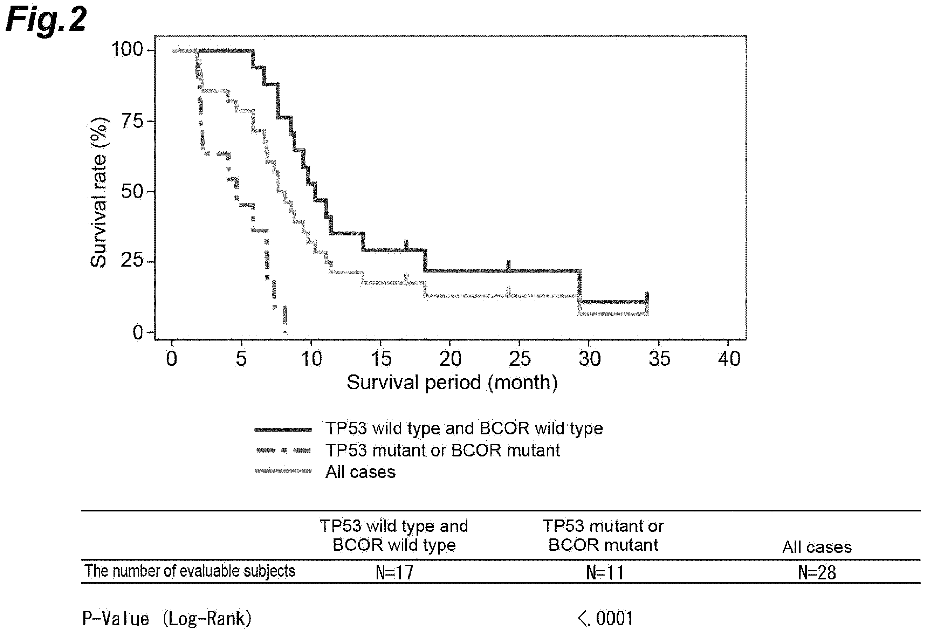

The patients described in Example 1 were divided into 17 cases of patients having TP53 wild type and BCOR wild type, and 11 cases of patients having TP53 mutant or BCOR mutant, and survival curves were compared. As shown in Figure 2, the median overall survival (mOS) of TP53 wild type and BCOR wild type tended to be long as compared with mOS of TP53 or BCOR mutant.

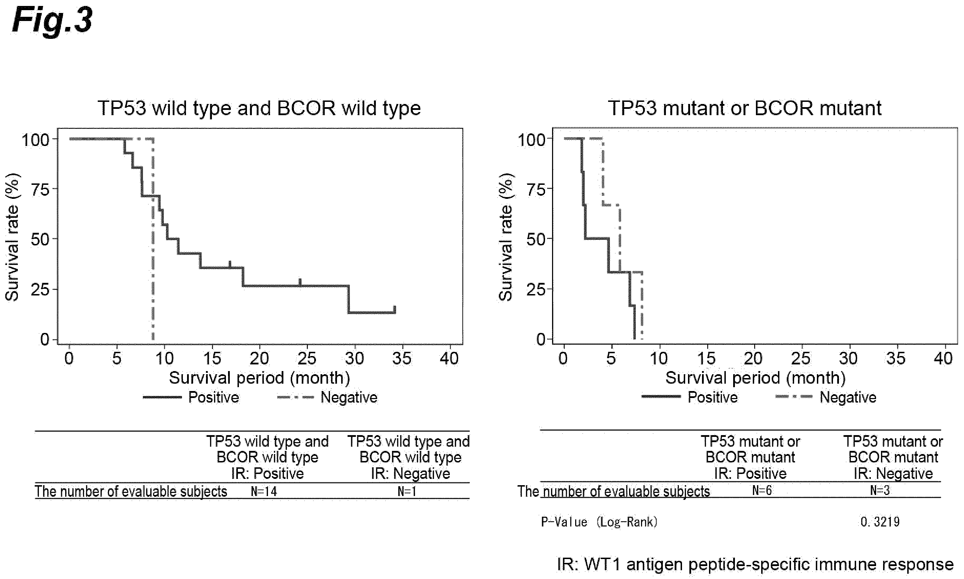

As for the 17 cases having TP53 wild type and BCOR wild type, and the 11 cases having TP53 mutant or BCOR mutant, WT1 antigen peptide-specific immune response induced by the WT1 peptide cocktail vaccine was confirmed by HLA tetramer assay mentioned later and delayed type hypersensitivity reaction. Results of comparing survival periods (OS) and immune response as to TP53 wild type and BCOR wild type, and TP53 mutant or BCOR mutant are shown in Figure 3. The results shown in Figure 3 are about 15 cases having TP53 wild type and BCOR wild type, and 9 cases having TP53 mutant or BCOR mutant except for 4 cases for which the determination of immune response was impossible.

It was shown for TP53 wild type and BCOR wild type that the overall survival tended to be long. Also, many cases positive to immune response were found in TP53 wild type and BCOR wild type (14 cases/15 cases). On the other hand, it was shown for TP53 mutant or BCOR mutant that the overall survival tended to be short, regardless of the positivity or negativity of immune response.

By this, it was demonstrated that the presence or absence of mutations in TP53 and BCOR gene is useful as gene markers for selecting potential patients with benefiting from treatment with the WT1 peptide vaccine.

### [Example 5: Usefulness of WT1 mRNA as gene marker - 1]

The expression of WT1 mRNA was confirmed as to 40 cases except for 2 cases which were unresponsive cases ascribable to the adverse reactions of azacytidine among the 42 cases of azacytidine unresponsive high-risk patients of Example 1.

### DNA extraction from peripheral blood and quality evaluation of specimen

Peripheral blood and bone marrow fluid were collected from the patients within 28 days before the start of administration of the WT1 peptide cocktail vaccine. The extraction and purification of RNA were performed from 7 mL of whole blood or 0.5 mL of bone marrow fluid using fully automatic RNA purification apparatus QIAcube (Qiagen N.V.). Reagents, tubes, etc. of RNeasy mini Kit (Qiagen N.V.), and a sample were loaded according to the QIAcube user manual. RNeasy-Mini- program was selected from QIAcube Standard Program stored in QIAcube, and RNA was extracted.

### Expression analysis of WT1 mRNA

The expression analysis of WT1 mRNA was conducted by using WT1 mRNA Measurement Kit II "Otsuka" (OTSUKA Pharmaceutical Co., Ltd.). Hereinafter, reagents attached to the kit were used, unless otherwise specified.

The concentration of the extracted RNA was adjusted to 50 ng/µL by adding RNase free water. A WT1/GAPDH mixed RNA standard solution was prepared as standard solution 1, standard solution 1 diluted 10-fold with a standard solution diluent was prepared as standard solution 2, and likewise, 10-fold dilution was repeated to prepare up to standard solution 5. A mixture of 10 µL of a mix for RT-PCR (R1) and 5 µL of a metal ion solution at this ratio per reaction was used as a reaction solution. A real-time PCR apparatus (Applied Biosystems 7500 Fast Dx, Applied Biosystems) was used, and RT-PCR reaction was performed according to "3. Measurement operation" in the protocol attached to the kit. The measurement values of WT1 mRNA and GSDPH mRNA in a sample were calculated by using a calibration curve prepared from the standard solutions 1 to 5.

The WT1 mRNA expression level was calculated according to "4. Method for calculating WT1 mRNA expression level" in the protocol attached to the kit as follows.

Specifically, as shown in the expression given below, the WT1 mRNA expression level was calculated by multiplying a value obtained by dividing the WT1 mRNA measurement value by the GAPDH mRNA measurement value (WT1 mRNA copy number per copy of GAPDH mRNA) by an average GAPDH mRNA copy number per µg of RNA in healthy adult humans (GAPDH mRNA expression level). 2.7 × 10⁷ (copies/µg RNA) is an average GAPDH mRNA measurement value per µg of RNA in healthy adult humans. $\begin{matrix}WT 1 mRNA \\ Expression level \\ \left(copies/μg RNA\right)\end{matrix} = \frac{WT 1 mRNA measurement value \left(copies/mL\right)}{GAPDH mRNA measurement value \left(copies/mL\right)} \times 2.7 \times {10}^{7} \left(copies/μg RNA\right)$

### Results (1)

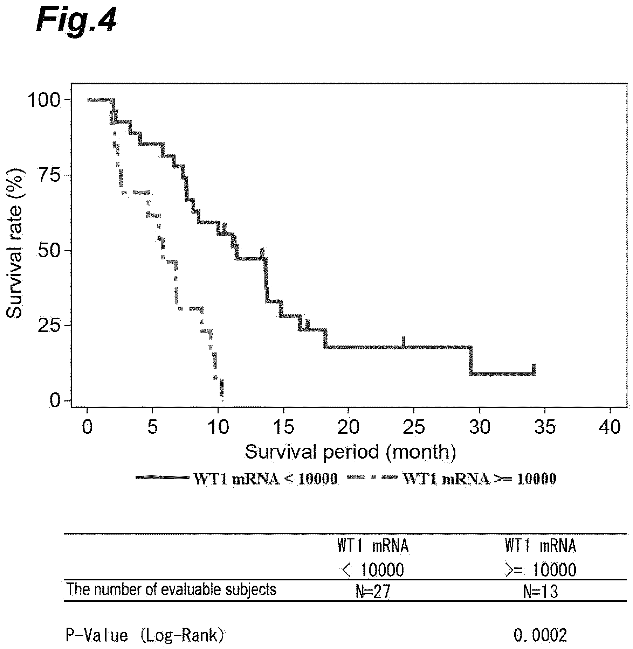

The results of the expression analysis of WT1 mRNA are shown in Figure 4. The expression of WT1 mRNA was found in peripheral blood in all cases among 40 cases. mOS of cases whose WT1 mRNA expression level was less than 10000 copies/µg RNA tended to be long as compared with mOS of cases whose WT1 mRNA expression level was 10000 copies/µg RNA or more.

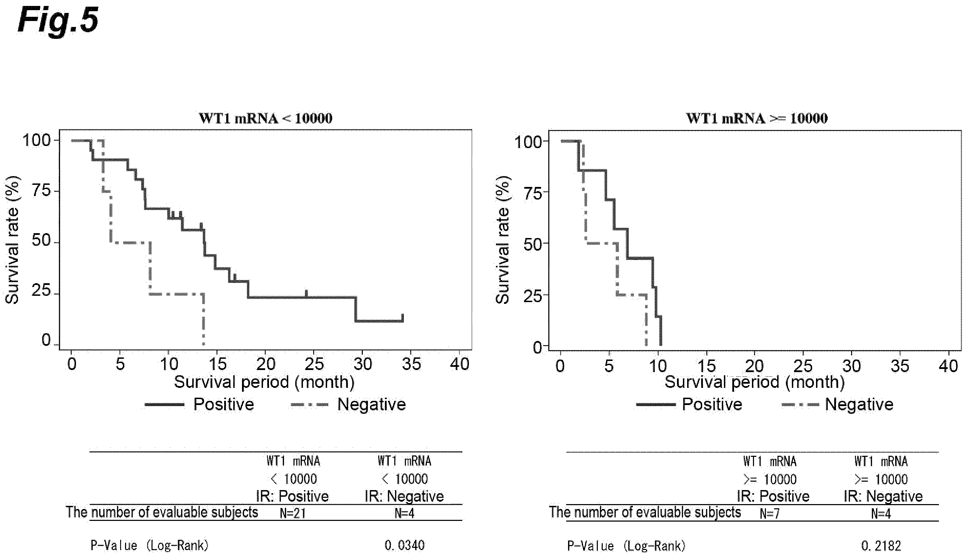

As for 27 cases whose WT1 mRNA expression level was less than 10000 copies/µg RNA, and 13 cases whose WT1 mRNA expression level was 10000 copies/µg RNA or more, WT1 antigen peptide-specific immune response was confirmed by HLA tetramer assay mentioned later and delayed type hypersensitivity reaction. Results of comparing survival periods (OS) and WT1 antigen peptide-specific immune response as to the expression level of WT1 mRNA are shown in Figure 5. The results shown in Figure 5 are about 25 cases whose WT1 mRNA expression level was less than 10000 copies/µg RNA, and 11 cases whose WT1 mRNA expression level was 10000 copies/µg RNA or more except for 4 cases for which the determination of WT1 antigen peptide-specific immune response was impossible.

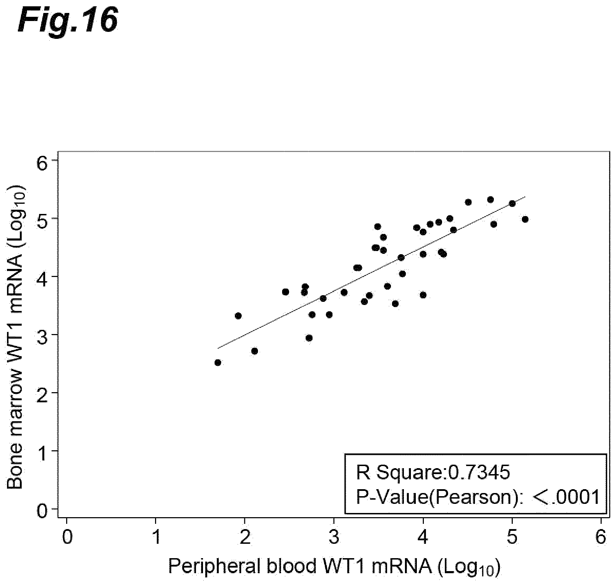

It was shown for the case group whose WT1 mRNA expression level was less than 10000 copies/µg RNA that OS of cases in which WT1 antigen peptide-specific immune response was found tended to be long. On the other hand, no difference in OS between the positivity and negativity of WT1 antigen peptide-specific immune response was seen in the case group whose WT1 mRNA expression level was 10000 copies/µg RNA or more. It was also confirmed that the WT1 mRNA expression level in peripheral blood and the WT1 mRNA expression level in bone marrow fluid had a correlation and similar tendencies were also seen in the bone marrow fluid (Figure 16).

### Results (2)

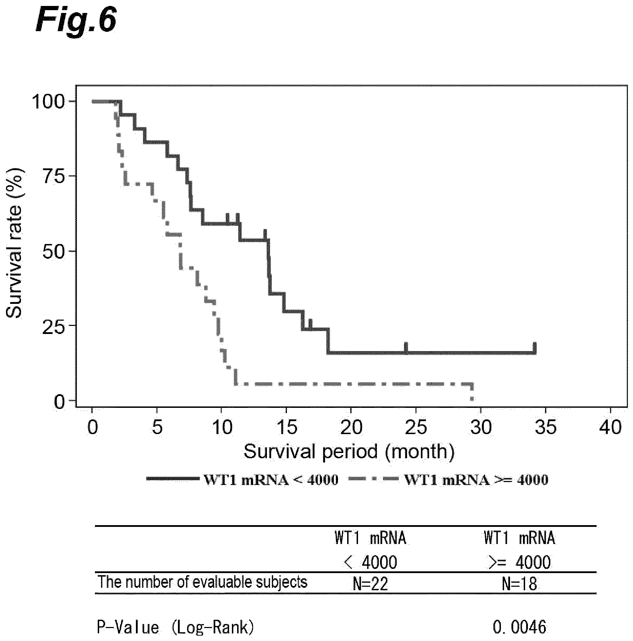

The results of the expression analysis of WT1 mRNA are shown in Figure 6. The expression of WT1 mRNA was found in peripheral blood in all cases among 40 cases. mOS of cases whose WT1 mRNA expression level was less than 4000 copies/µg RNA tended to be long as compared with mOS of cases whose WT1 mRNA expression level was 4000 copies/µg RNA or more.

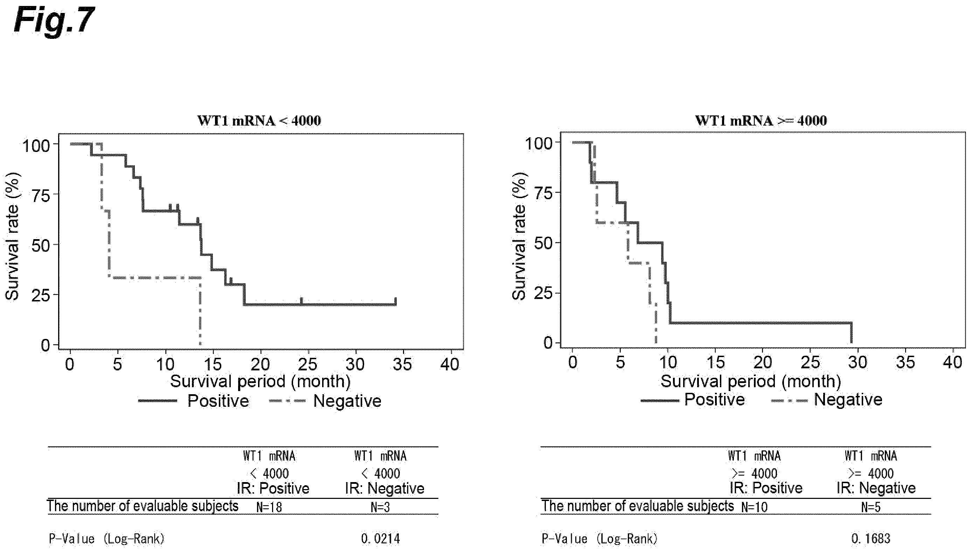

As for 22 cases whose WT1 mRNA expression level was less than 4000 copies/µg RNA, and 18 cases whose WT1 mRNA expression level was 4000 copies/µg RNA or more, WT1 antigen peptide-specific immune response was confirmed by HLA tetramer assay mentioned later and delayed type hypersensitivity reaction. Results of comparing survival periods (OS) and WT1 antigen peptide-specific immune response as to the expression level of WT1 mRNA are shown in Figure 7. The results shown in Figure 7 are about 21 cases whose WT1 mRNA expression level was less than 4000 copies/µg RNA, and 15 cases whose WT1 mRNA expression level was 4000 copies/µg RNA or more except for 4 cases for which the determination of WT1 antigen peptide-specific immune response was impossible.

It was shown for the case group whose WT1 mRNA expression level was less than 4000 copies/µg RNA that OS of cases in which WT1 antigen peptide-specific immune response was found tended to be long. On the other hand, no difference in OS between the positivity and negativity of WT1 antigen peptide-specific immune response was seen in the case group whose WT1 mRNA expression level was 4000 copies/µg RNA or more. It was also confirmed that the WT1 mRNA expression level in peripheral blood and the WT1 mRNA expression level in bone marrow fluid had a correlation and similar tendencies were also seen in the bone marrow fluid (Figure 16).

By this, it was demonstrated that the expression level of WT1 mRNA is useful as a gene marker for selecting potential patients with benefiting treatment with the WT1 peptide vaccine. It was also demonstrated that both 10000 copies/µg RNA and 4000 copies/µg RNA are useful as the reference values of the marker.

### [Example 6: Detection of WT1 antigen peptide-specific immune response by HLA tetramer assay]

The measurement of the ratio of WT1 antigen peptide-specific CD8 T cells among lymphocytes or among CD8-positive lymphocytes was carried out by the flow cytometry method using a tetramer reagent as to blood collected from patients before and after administration of the WT1 peptide cocktail vaccine.

### Administration and blood collection

A test was carried out as to the 42 cases of azacytidine unresponsive high-risk patients of Example 1. The breakdown of the 42 cases regarding HLA type was 10 cases of HLA-A*02:01- or HLA-A*02:06-positive myelodysplastic syndrome (MDS) patients, 25 cases of HLA-A*24:02-positive MDS patients, 5 cases of HLA-A"02:01-positive and HLA-A*24:02-positive MDS patients, and 2 cases of HLA-A*02:06-positive and HLA-A*24:02-positive MDS patients. The collection of peripheral blood from the patients and assay were performed within 28 days before the start of administration of the pharmaceutical composition, on 15 days after the 2nd administration, on 15 days after the 6th administration, on 15 days after the 12th administration, on 15 days after the 18th administration, subsequently on 15 days after administration every 6 doses, and within 28 days after the final administration. For the administration, 1.75 mg, 3.5 mg or 10.5 mg of the WT1 peptide cocktail vaccine per dose was intradermally administered to a patient.

### HLA tetramer reagent

Since WT1 antigen peptide-specific CD8 T cells have HLA restriction, measurement was performed by using a tetramer reagent compatible with HLA of the patients. A tetramer of fluorescent dye phycoerythrin (PE)-labeled HLA-A*24:02 (T-Select HLA-A*24:02 WT1(mutant)Tetramer-CYTWNQMNL PE-labeled, Medical & Biological Laboratories Co., Ltd.) prepared by using a peptide consisting of CYTWNQMNL (SEQ ID NO: 4) of the WT1 protein was used for the HLA-A*24:02-positive patients. Hereinafter, this reagent is also referred to as "PE-labeled WT1 2402".

A tetramer of fluorescent dye allophycocyanin (APC)-labeled HLA-A*02:01 (T-Select HLA-A*02:01 WT1₁₂₆₋₁₃₄ Tetramer-RMFPNAPYL-APC, Medical & Biological Laboratories Co., Ltd., capable of detecting both HLA-A*02:01 and HLA-A*02:06) prepared by using a peptide consisting of RMFPNAPYL (SEQ ID NO: 2) of the WT1 protein and a tetramer of HLA-A*02:01 in which a peptide consisting of VLDFAPPGA (SEQ ID NO: 9) of the WT1 protein was labeled with a fluorescent dye phycoerythrin (PE) (HLA-A*02:01 Tetramer-VLDFAPPGA-PE, commissioned production by Medical & Biological Laboratories Co., Ltd., capable of detecting both HLA-A*02:01 and HLA-A*02:06) were used for the HLA-A*02:01- or HLA-A*02:06-positive patients. Hereinafter, the former reagent is also referred to as "APC-labeled WT1 0201", and the latter reagent is also referred to as "PE-labeled WT1 0201". The tetramer reagents were placed in microtubes before use and centrifuged at 1620 × g at room temperature for 5 minutes, and the supernatants were used.

### Staining

The staining of the patient subjects having HLA-A*24:02 was performed as follows.

The collected blood was dispensed in 2.5 mL into a tube for a sample and in the remaining amount into a tube for a control. To the tube for a sample, 5 µL of PE-labeled WT1 2402 was added. After reaction at room temperature for 10 minutes in the dark, 15 µL of FITC-labeled CD8 (CytoStat/Coulter Clone T8-FITC, Beckman Coulter K.K.), and 12.5 µL each of 7-AAD (7-AAD Staining Solution, Nippon Becton Dickinson Co., Ltd.), PC5-labeled CD4 (IO Test CD4-PC5, Beckman Coulter K.K.), and PC5-labeled CD19 (IO Test CD19-PC5, Beckman Coulter K.K.) were added to the tube for a sample, and stirred. After reaction at room temperature for 15 minutes, 26 mL of a lysing solution preparation solution (BD FACSTM Lysing solution (Nippon Becton Dickinson Co., Ltd.) diluted 10-fold with purified water) was added thereto, then stirred, left standing at room temperature for 10 minutes, and then centrifuged at 300 × g for 5 minutes. The supernatant was removed by suction with an aspirator, and after stirring, 30 mL of PBS with azide (PBS containing 1% sodium azide) was dispensed into each tube and centrifuged at 300 × g for 5 minutes. After removal of the supernatant by suction with an aspirator, the resultant was resuspended in 300 µL of a cell fix preparation solution (BD FACS Cell Fix, Nippon Becton Dickinson Co., Ltd.) and transferred to a tube for measurement.

The staining of the patient subjects having HLA-A*02:01 or HLA-A*02:06 was performed as follows.

The collected blood was dispensed in 2.5 mL into a tube for a sample and in the remaining amount into a tube for a control. To the tube for a sample, 5 µL each of APC-labeled WT1 0201 and PE-labeled WT1 0201 was added. After reaction at room temperature for 10 minutes in the dark, 15 µL of FITC-labeled CD8, and 12.5 µL each of 7-AAD, APC-H7-labeled CD3 (CD3 APC-H7-labeled, Nippon Becton Dickinson Co., Ltd.), Pacific Blue-labeled CD4 (IOTest CD4-Pacific Blue, Beckman Coulter K.K.), and PE-Cy7-labeled CD19 (IOTest CD19-PC7, Beckman Coulter K.K.) were added to the tube for a sample, and stirred. After reaction at room temperature for 15 minutes, 26 mL of a lysing solution preparation solution was added thereto and then thoroughly stirred. The resultant was left standing at room temperature for 10 minutes and then centrifuged at 300 × g for 5 minutes. The supernatant was removed by suction with an aspirator, and after stirring, 30 mL of PBS with azide was dispensed into each tube and centrifuged at 300 × g for 5 minutes. After removal of the supernatant by suction with an aspirator, the resultant was resuspended in 300 µL of a cell fix preparation solution and transferred to a tube for measurement.

The staining of the patient subjects having both HLA-A*24:02 and HLA-A*02:01 or HLA-A*02:06 was performed by both the operations of the staining of the patient subjects having HLA-A*24:02 and the staining of the patient subjects having HLA-A*02:01 or HLA-A*02:06.

### Flow cytometry analysis

The measurement of the ratio of WT1 antigen peptide-specific CD8 T cells among lymphocytes or among CD8-positive lymphocytes was carried out by the flow cytometry method.

The samples prepared as described above were measured in a flow cytometer FACS Canto II (BD Biosciences). In the flow cytometer, scattered light (forward scattered light (FSC) which reflects a size and side scattered light (SSC) which reflects an internal structure) having intensity according to the characteristics (size and internal structure) of cells, and fluorescence depending on the amount of a labeled antibody bound are generated. The intensity of these parameters was measured as to individual cells in the samples. A particular population can be calculated by converting the distribution of the individual cells into a graph on the basis of the obtained intensity of the parameters. Lymphocyte fractions that corresponded to lymphocytes from the size and internal structure of the cells and were negative to anti-CD4 antibody/anti-CD19 antibody/7-AAD staining solution and positive only to the anti-CD8 antibody (patient subjects having HLA-A*02:01 or HLA-A*02:06) or positive to both the anti-CD3 antibody and the anti-CD8 antibody (patient subjects having HLA-A*02:01/06) were extracted, and a tetramer-positive/lymphocyte fraction or a tetramer-positive/CD8-positive cell fraction was evaluated in the fractions. A gate for an FITC-labeled CD8-positive and PE-labeled WT1 2402-positive cell population was set with 10³ of the y-axis as a guideline (gate CD8(+)tet(+)). A gate for an FITC-labeled CD8-positive and PE-labeled WT1 0201-positive cell population was set with 3 x 10² of the y-axis as a guideline (gate CD8(+)tet(+)). A gate for an FITC-labeled CD8-positive and APC-labeled WT1 0201-positive cell population was set with 3 x 10² of the y-axis as a guideline (gate CD8(+)tet(+)).

For the HLA-A*24:02 patients, in the case that an event was found in the gate CD8(+)tet(+) before administration of the WT1 peptide cocktail vaccine, the ratios of PE-labeled WT1 24:02-positive cells with strongly CD8-positive lymphocytes before and after administration of the WT1 peptide cocktail vaccine as denominators were compared. In the case that no event was found in the gate CD8(+)tet(+) before administration, the number of events in the gate CD8(+)tet(+) was calculated after administration. For the HLA-A*02:01 or HLA-A*02:06 patients, in the case that an event was found in the gate CD8(+)tet(+) before administration of the WT1 peptide cocktail vaccine, the ratios of PE-labeled WT1 02:01 or 02:06-positive cells and APC-labeled WT1 02:01 or 02:06-positive cells with strongly CD8-positive lymphocytes before and after administration of the WT1 peptide cocktail vaccine as denominators were compared. In the case that no event was found in the gate CD8(+)tet(+) before administration, the number of events in the gate CD8(+)tet(+) was calculated after administration. A reference for determining whether WT1 antigen peptide-specific immune response was positive or negative by the HLA tetramer assay was set as follows. In the case that positivity or maintenance was determined at any point in time after administration, determination by the HLA tetramer assay was positivity or maintenance. In the case that negativity was determined at all points in time after administration, determination by the HLA tetramer assay was negativity.

**[Table 3]**

| In the case that cells were not found in CD8⁺tet⁺ gate before administration | | |
|---|---|---|
| CD8⁺tet⁺ | tet⁺/CD8⁺ after administration with respect to before administration | Determination by tetramer assay |
| ≥ 10 | - | Positivity |
| < 10 | - | Negativity |

| In the case that cells were found in CD8⁺tet⁺gate before administration | | |
|---|---|---|
| CD8⁺tet⁺ | tet⁺/CD8⁺ after administration with respect to before administration | Determination by tetramer assay |
| ≥ 10 | ≥ 2 times | Positivity |
| ≥ 10 | More than 1/2 times - less than 2 times | Maintenance |
| ≥ 10 | 1/2 times ≥ | Negativity |
| < 10 | - | Negativity |

### Results

As a result of performing determination as to all of the 47 cases on the basis of the reference, 30 cases (63.8%) were determined as being positive in the determination by the HLA tetramer assay, 3 cases (6.4%) were determined as being maintained in the determination by the HLA tetramer assay, and 13 cases (27.7%) were determined as being negative in the determination by the HLA tetramer assay. As for 1 case (2.1%), the determination by the HLA tetramer assay was impossible.

### [Example 7: Detection of delayed hypersensitivity reaction (DTH reaction)]

### Preparation of drug solution for DTH

2 mL of injectable water and 1.8 mL of injectable water were added to 20 mg of the WT1 killer peptide conjugate and 18 mg of the WT1 helper peptide, respectively, of the WT1 peptide cocktail vaccine shown in Example 1 to prepare a WT1 killer peptide conjugate solution (10 mg/mL) and a WT1 helper peptide solution (10 mg/mL). Further, each was diluted 10-fold (1 mg/mL) using a lactated Ringer's solution. The drug solutions for DTH were used within 3 hours after preparation.

### Inoculation and measurement

100 µL (100 µg) each of two types of drug solutions for DTH (WT1 killer peptide conjugate solution and WT1 helper peptide solution) was intradermally injected 3 cm or more apart to the forearm of a patient. As a negative control, 100 µL of a lactated Ringer's solution (control solution) was intradermally injected to the forearm on the same side 3 cm or more distant from the administration sites of the drug solutions for DTH. Two days after administration, redness diameters [major axis, minor axis, and properties (double redness, induration, ulcer, etc.)] were measured. The difference of the major axis of redness of the control solution from the major axis of redness of the WT1 killer peptide conjugate solution or the WT1 helper peptide solution was calculated, and DTH reaction was scored from the difference according to the following table.

**[Table 4]**

| Redness diameter difference | Score |
|---|---|
| Less than 2 mm | 0 |
| 2 mm or more and less than 5 mm | +/- |
| 5 mm or more and less than 10 mm | 1 |
| 10 mm or more and less than 15 mm | 2 |
| More than 15 mm | 3 |

The DTH test was conducted before the start of administration of the WT1 peptide cocktail vaccine (within 28 days before administration), on 2 days after the 2nd administration, on 2 days after the 12th administration, and after the final administration (within 28 days after administration). Scores of DTH reaction were determined on the basis of the reference. The maximum score in each case after administration was subjected to the subsequent analysis.

### Results

As a result of determining scores as to all of the 47 cases on the basis of the reference, 11 cases (23.4%) were given score 0, 4 cases (8.5%) were given score +/-, 9 cases (19.1%) were given score 1, 4 cases (8.5%) were given score 2, 9 cases (19.1%) were given score 3, patients for which the determination of scores was impossible were 7 cases (14.9%), and patients for which the DTH test were not able to be carried out were 3 cases (6.4%).

### [Example 8: Establishment of reference for classifying positivity or negativity of WT1 antigen peptide-specific immune response]

As for WT1 antigen peptide-specific immune response induced by the WT1 peptide cocktail vaccine, a reference for classifying the positivity or negativity of WT1 antigen peptide-specific immune response was established by bi-directionally analyzing determination results by the HLA tetramer assay, and the maximum score of the DTH test using the WT1 killer peptide conjugate.

Since this Example was aimed at the comprehensive determination of WT1 antigen peptide-specific immune response, a total of 47 cases, 42 high-risk cases as well as 5 low-risk cases described in Example 1, were used as analysis subjects. However, among them, 1 case for which the determination by the HLA tetramer assay was impossible in Example 6, and 10 cases for which the determination of DTH scores was impossible or for which the DTH test were not able to be carried out in Example 7 were excluded from this analysis.

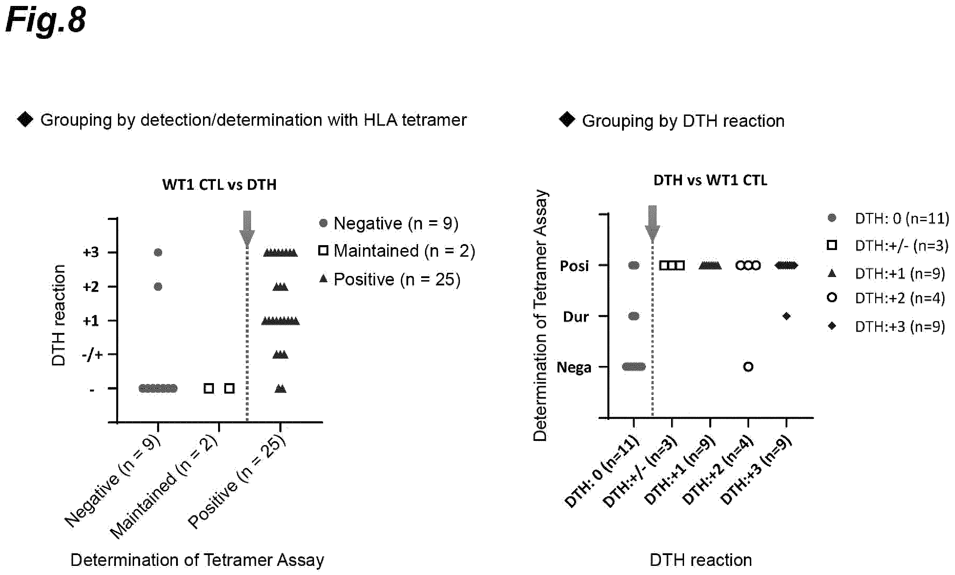

It was found that most of cases determined as being positive from determination results by the HLA tetramer assay also had a score of +/- or more in the determination of the DTH test using the WT1 killer peptide conjugate (left diagram of Figure 8). It was also found that most of cases having a score of less than +/- in the determination of the DTH test using the WT1 killer peptide conjugate were determined as being negative in the HLA tetramer assay (right diagram of Figure 8). From these results, the positions indicated by the respective arrows in the right diagram and left diagram of Figure 8 were established as WT1 antigen peptide-specific immune response-positive references.

Specifically, in the case that the determination results by the HLA tetramer assay are positivity or the determination of the DTH test using the WT1 killer peptide conjugate is a score of +/- or more (difference from a control is 2 mm or more), it can be determined that the WT1 antigen peptide-specific immune response caused by the WT1 peptide cocktail vaccine is positive. On the other hand, in the case that the determination results by the HLA tetramer assay are maintenance or negativity and the determination of the DTH test using the WT1 killer peptide conjugate is 0 (difference from a control is less than 2 mm), it can be determined that the WT1 antigen peptide-specific immune response caused by the WT1 peptide cocktail vaccine is negative.

When all of the 47 cases were classified by the reference, 33 cases (70.2%) were determined as being positive to WT1 antigen peptide-specific immune response and 9 cases (19.1%) were determined as being negative to WT1 antigen peptide-specific immune response. As for 5 cases, determination was impossible because the DTH test was not carried out or internal bleeding was observed due to the DTH test; thus WT1 antigen peptide-specific immune response was unknown (10.7%).

### [Example 9: Analysis of WT1 antigen peptide-specific immune response and clinical effect]

Relationship with a clinical effect was analyzed as follows as to a total of 36 cases, 28 cases determined as being positive to WT1 antigen peptide-specific immune response and 8 cases determined as being negative thereto according to the reference of Example 8, among the 42 cases of azacytidine unresponsive high-risk patients described in Example 1. 2 cases which were unresponsive cases ascribable to the adverse reactions of azacytidine, and 4 cases for which the determination of WT1 antigen peptide-specific immune response was impossible because the DTH test was not carried out or internal bleeding was observed due to the DTH test were excluded from the analysis.

### Change in myeloblast

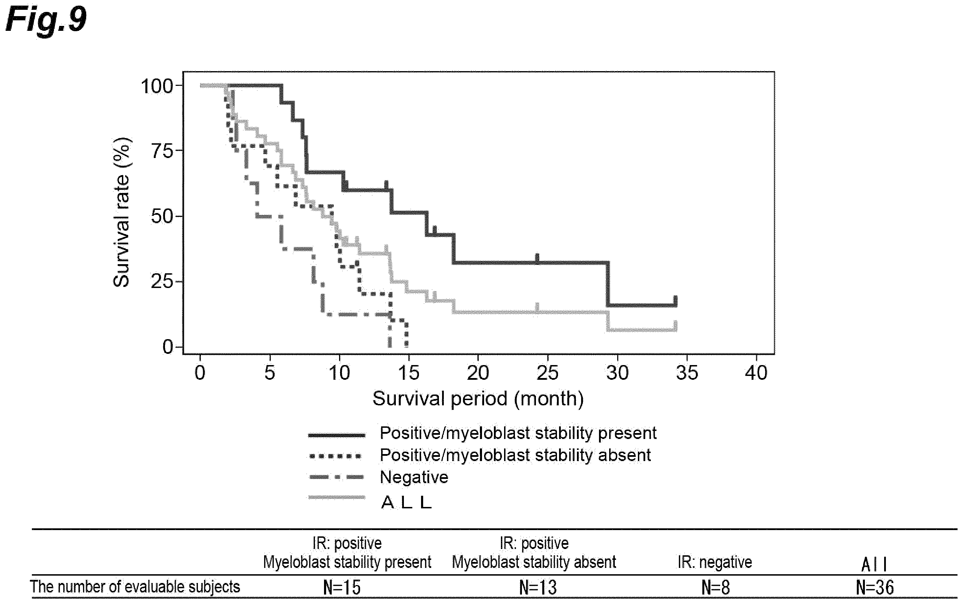

WT1 antigen peptide-specific immune response and change in myeloblasts were analyzed. In the case that change, from a Pre value, in the ratio of myeloblasts shifted with 150% or less at two points in time or more up to approximately 3 months after initial administration (bone marrow fluid was collected approximately 15 days after the 2nd administration and/or approximately 15 days after the 6th administration in administration once 2 weeks), it was determined that gemmule stabilization was present. In the case that change, from the Pre value, in the ratio of myeloblasts was more than 150%, it was determined that gemmule stabilization was absent (exacerbation). As shown in Figure 9, many cases in which gemmules were stabilized for a long period were found in the case group positive to WT1 antigen peptide-specific immune response.

### Transition period to AML

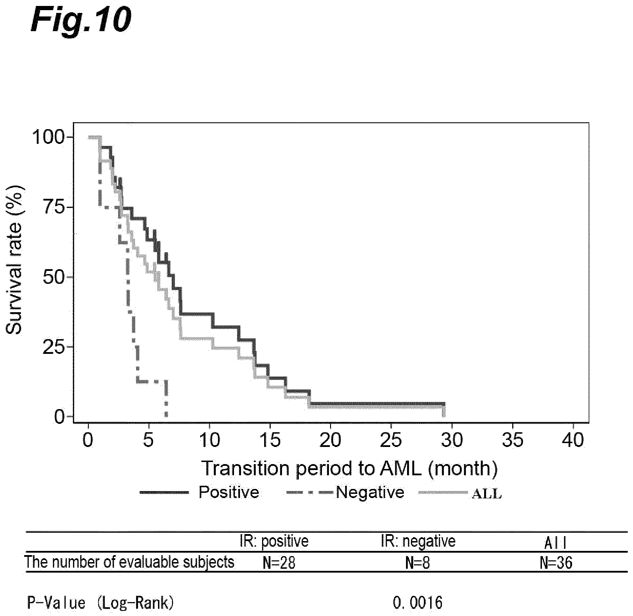

Transition periods to acute myeloid leukemia (AML) based on the positivity or negativity of WT1 antigen peptide-specific immune response were compared. As shown in Figure 10, the transition period to AML tended to be long in the case group positive to WT1 antigen peptide-specific immune response.

### Survival curve

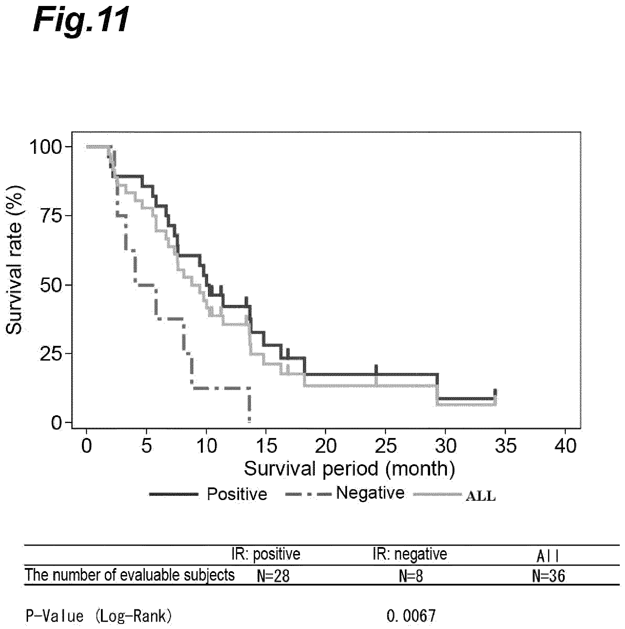

Survival curves based on the positivity or negativity of WT1 antigen peptide-specific immune response were compared. As shown in Figure 11, mOS of 28 cases determined to be positive to WT1 antigen peptide-specific immune response tended to be long as compared with mOS of 8 cases determined to be negative to WT1 antigen peptide-specific immune response.

### Myeloblast stabilization and survival curve

As shown in Figure 9, cases in which the stabilization of myeloblasts was found were absent in 8 cases determined to be negative to WT1 antigen peptide-specific immune response. On the other hand, in 28 cases determined to be positive to WT1 antigen peptide-specific immune response, the stabilization of myeloblasts was found in 15 cases (53.6%). mOS of the cases that were positive to WT1 antigen peptide-specific immune response and were found to have the stabilization of myeloblasts tended to be longest.

### Karyotype and survival curve

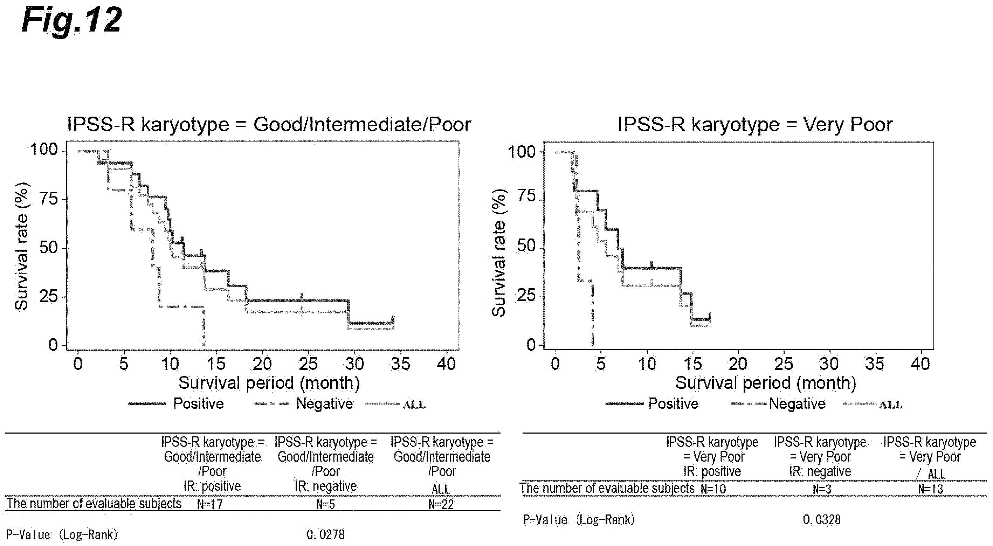

35 cases except for 1 case whose karyotype was unknown were classified on IPSS-R karyotype basis, and WT1 antigen peptide-specific immune response and survival curves were compared. As shown in Figure 12, it was found for good/intermediate/poor groups that mOS of a case group determined as being positive to WT1 antigen peptide-specific immune response tended to be long as compared with a case group determined as being negative to WT1 antigen peptide-specific immune response. Likewise, in a poor-prognosis group with karyotype being very poor, mOS of a case group determined as being positive to WT1 antigen peptide-specific immune response tended to be longer than that of a case group determined as being negative to WT1 antigen peptide-specific immune response.

### Results

Many cases in which myeloblasts were stabilized were found and the prolongation of the transition period to AML was found in the case group determined as being positive to WT1 antigen peptide-specific immune response as compared with the case group determined as being negative to WT1 antigen peptide-specific immune response. Also, the tendency to prolong mOS was shown. The possibility was suggested that WT1 antigen peptide-specific immune response was induced, whereby myeloblasts were stabilized and in association with this, the prolongation of a transition period to AML and a survival period was found. In the case of conducting stratified analysis on a good-prognosis case group and a poor-prognosis case group of karyotype, the tendency to prolong mOS was also seen.

### [Example 10: Analysis by sex difference]

The median survival time of each sex difference was compared with historical data and the median survival time of BSC of a rigosertib test, as to 40 cases except for 2 cases which were unresponsive cases ascribable to the adverse reactions of azacytidine among the 42 cases of azacytidine unresponsive high-risk patients of Example 1.

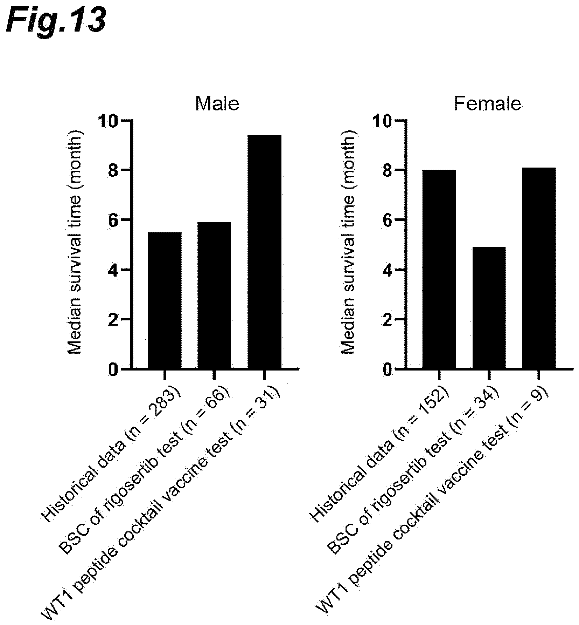

As shown in Table 5, many cases determined as being positive to WT1 antigen peptide-specific immune response were found in males. As a result of comparison with historical data (Prebet et al., Journal of Clinical Oncology 29, no. 24, 3322-3327) and a control (BSC of the rigosertib test) in the ONTIME test of rigosertib (Garcia-Manero et al., The Lancet Oncology 17, no. 4, p. 496-508 (2016)), as shown in Figure 13, the WT1 peptide cocktail vaccine was found to prolong OS in males rather than females, suggesting the possibility that the effect was high.

**[Table 5]**

| WT1 peptide cocktail vaccine test | Male | Female | Total |
|---|---|---|---|
| The number of cases (%) | 31 (77.5%) | 9 (22.5%) | 40 |
| Immune response | Positive: 26 (83.9%) | Positive: 2 (22.2%) | Positive: 28 (70%) |
| | Negative: 4 (12.9%) | Negative: 4 (44.4%) | Negative: 8 (20%) |
| | Impossible determination: 1 (3.2%) | Impossible determination: 3 (33.3%) | Impossible determination: 4 (10%) |

### [Example 11: Usefulness of WT1 mRNA as gene marker - 2]

The expression of WT1 mRNA was confirmed as to 40 cases except for 2 cases which were unresponsive cases ascribable to the adverse reactions of azacytidine among the 42 cases of azacytidine unresponsive high-risk patients of Example 1.

### DNA extraction from peripheral blood and quality evaluation of specimen

Peripheral blood and bone marrow fluid were collected from the patients within 28 days before the start of administration of the WT1 peptide cocktail vaccine. The extraction and purification of RNA were performed from 7 mL of whole blood or 0.5 mL of bone marrow fluid using fully automatic RNA purification apparatus QIAcube (Qiagen N.V.). Reagents, tubes, etc. of RNeasy mini Kit (Qiagen N.V.), and a sample were loaded according to the QIAcube user manual. RNeasy-Mini- program was selected from QIAcube Standard Program stored in QIAcube, and RNA was extracted.

### Expression analysis of WT1 mRNA

The expression analysis of WT1 mRNA was conducted by using WT1 mRNA Measurement Kit II "Otsuka" (OTSUKA Pharmaceutical Co., Ltd.). Hereinafter, reagents attached to the kit were used, unless otherwise specified.

The concentration of the extracted RNA was adjusted to 50 ng/µL by adding RNase free water. A WT1/GAPDH mixed RNA standard solution was prepared as standard solution 1, standard solution 1 diluted 10-fold with a standard solution diluent was prepared as standard solution 2, and likewise, 10-fold dilution was repeated to prepare up to standard solution 5. A mixture of 10 µL of a mix for RT-PCR (R1) and 5 µL of a metal ion solution at this ratio per reaction was used as a reaction solution. A real-time PCR apparatus (Applied Biosystems 7500 Fast Dx, Applied Biosystems) was used, and RT-PCR reaction was performed according to "3. Measurement operation" in the protocol attached to the kit. The measurement values of WT1 mRNA and GSDPH mRNA in a sample were calculated by using a calibration curve prepared from the standard solutions 1 to 5.

The WT1 mRNA expression level was calculated according to "4. Method for calculating WT1 mRNA expression level" in the protocol attached to the kit as follows.

Specifically, as shown in the expression given below, the WT1 mRNA expression level was calculated by multiplying a value obtained by dividing the WT1 mRNA measurement value by the GAPDH mRNA measurement value (WT1 mRNA copy number per copy of GAPDH mRNA) by an average GAPDH mRNA copy number per µg of RNA in healthy adult humans (GAPDH mRNA expression level). 2.7 × 10⁷ (copies/µg RNA) is an average GAPDH mRNA measurement value per µg of RNA in healthy adult humans. $\begin{matrix}WT 1 mRNA \\ Expression level \\ \left(copies/μg RNA\right)\end{matrix} = \frac{WT 1 mRNA measurement value \left(copies/mL\right)}{GAPDH mRNA measurement value \left(copies/mL\right)} \times 2.7 \times {10}^{7} \left(copies/μg RNA\right)$

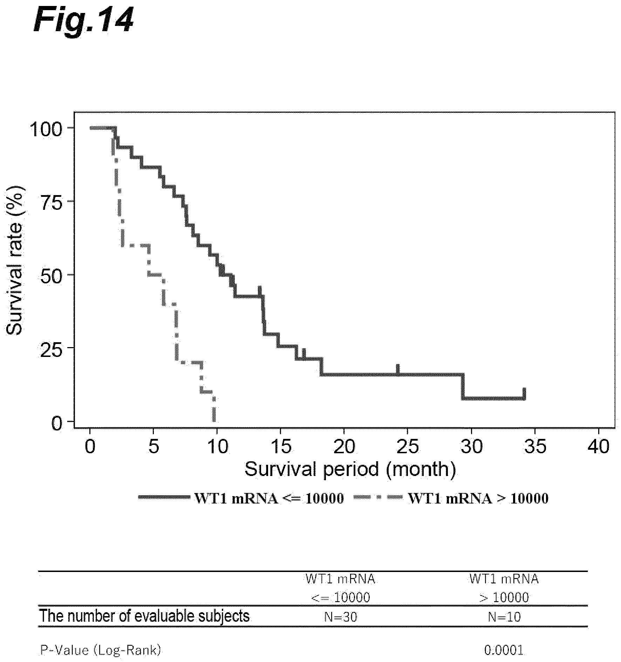

The results of the expression analysis of WT1 mRNA are shown in Figure 14. The expression of WT1 mRNA was found in peripheral blood in all cases among 40 cases. mOS of cases whose WT1 mRNA expression level was 10000 copies/µg RNA or less tended to be long as compared with mOS of cases whose WT1 mRNA expression level was higher than 10000 copies/µg RNA.

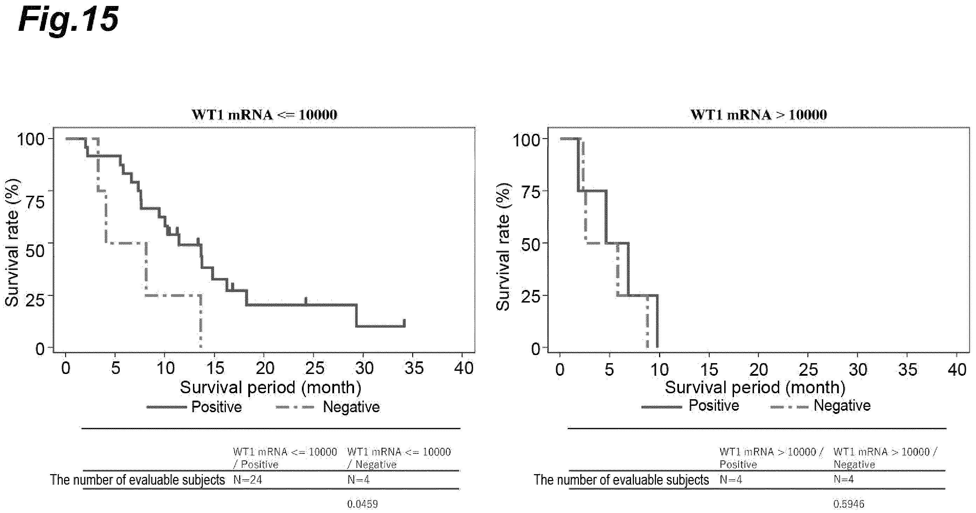

As for 30 cases whose WT1 mRNA expression level was 10000 copies/µg RNA or less, and 10 cases whose WT1 mRNA expression level was higher than 10000 copies/µg RNA, WT1 antigen peptide-specific immune response was confirmed by HLA tetramer assay mentioned later and delayed type hypersensitivity reaction. Results of comparing survival periods (OS) and WT1 antigen peptide-specific immune response as to the expression level of WT1 mRNA are shown in Figure 15. The results shown in Figure 15 are about 28 cases whose WT1 mRNA expression level was 10000 copies/µg RNA or less, and 8 cases whose WT1 mRNA expression level was higher than 10000 copies/µg RNA except for 4 cases for which the determination of WT1 antigen peptide-specific immune response was impossible.

By this, it was demonstrated that the expression level of WT1 mRNA is useful as a gene marker for selecting potential patients with benefiting treatment with the WT1 peptide vaccine. It was also demonstrated that 10000 copies/µg RNA is useful as the reference value of the marker.

It was shown for the case group whose WT1 mRNA expression level was 10000 copies/µg RNA or less that OS of cases in which WT1 antigen peptide-specific immune response was found tended to be long. On the other hand, no difference in OS between the positivity and negativity of WT1 antigen peptide-specific immune response was seen in the case group whose WT1 mRNA expression level was higher than 10000 copies/µg RNA. It was also confirmed that the WT1 mRNA expression level in peripheral blood and the WT1 mRNA expression level in bone marrow fluid had a correlation and similar tendencies were also seen in the bone marrow fluid (Figure 16).

## Claims

1. A method for selecting a potential subject with benefiting from a pharmaceutical composition for treating or preventing cancer, comprising:
determining the presence or absence of a mutation in tumor protein p53 (TP53) gene and BCL6 co-repressor (BCOR) gene by using a sample of body fluid collected from the subject; and
providing an indication that the subject is a potential subject with benefiting from the pharmaceutical composition in the case of TP53 wild type and BCOR wild type, wherein the pharmaceutical composition comprises a peptide comprising an amino acid sequence selected from the group consisting of RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7), VLDFAPPGA (SEQ ID NO: 9), CMTWNQMNL (SEQ ID NO: 3) and CYTWNQMNL (SEQ ID NO: 4) or a pharmaceutically acceptable salt thereof,
the cancer is selected from the group consisting of acute myeloid leukemia (AML), myelodysplastic syndrome (MDS), myeloproliferative neoplasm (MPN), chronic myeloid leukemia (CML), chronic myelomonocytic leukemia (CMML), and juvenile myelomonocytic leukemia (JMML),
the TP53 wild type refers to the case that no mutation is present in the TP53 gene, or the case that the TP53 gene, even if a mutation is present, does not lose an original function or is not accompanied by abnormality, and
the BCOR wild type refers to the case that no mutation is present in the BCOR gene, or the case that the BCOR gene, even if a mutation is present, does not lose an original function or is not accompanied by abnormality.

2. The method according to claim 1, wherein the pharmaceutical composition comprises
a peptide comprising an amino acid sequence selected from the group consisting of RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7) and VLDFAPPGA (SEQ ID NO: 9), and
a peptide comprising an amino acid sequence selected from the group consisting of CMTWNQMNL (SEQ ID NO: 3) and CYTWNQMNL (SEQ ID NO: 4) or a pharmaceutically acceptable salt thereof.

3. The method according to claim 1 or 2, wherein the pharmaceutical composition comprises a compound represented by the formula (1): 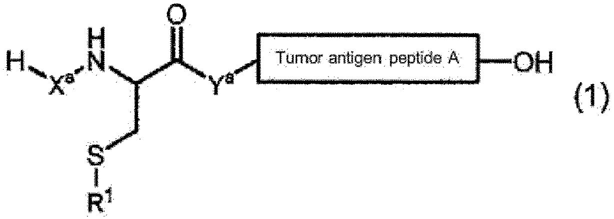 wherein X^{a} and Y^{a} represent a single bond, tumor antigen peptide A represents a peptide consisting of any amino acid sequence selected from among the following amino acid sequences:
RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7) and VLDFAPPGA (SEQ ID NO: 9),
the amino group of the N-terminal amino acid of the tumor antigen peptide A is bonded to Y^{a} in the formula (1), the carbonyl group of the C-terminal amino acid of the tumor antigen peptide A is bonded to the hydroxy group in the formula (1), R¹ represents a hydrogen atom or tumor antigen peptide B,
the tumor antigen peptide B differs in sequence from the tumor antigen peptide A and represents a peptide consisting of any amino acid sequence selected from among the following amino acid sequences: CMTWNQMNL (SEQ ID NO: 3) and CYTWNQMNL (SEQ ID NO: 4), and the thioether group of the cysteine residue of the tumor antigen peptide B is bonded to the thioether group in the formula (1), or a pharmaceutically acceptable salt thereof.

4. The method according to claim 3, wherein the compound represented by the formula (1) is a compound represented by the formula (2): 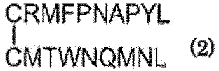
wherein the bond between C and C represents a disulfide bond,
or a pharmaceutically acceptable salt thereof.

5. The method according to claim 3, wherein the compound represented by the formula (1) is a compound represented by the formula (3): 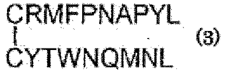
wherein the bond between C and C represents a disulfide bond,
or a pharmaceutically acceptable salt thereof.

6. The method according to any one of claims 1 to 5, wherein the pharmaceutical composition further comprises a peptide comprising an ammo acid sequence selected from the group consisting of
CNKRYFKLSHLQMHSRK (SEQ ID NO: 11),
CNKRYFKLSHLQMHSRKH (SEQ ID NO: 12),
CNKRYFKLSHLQMHSRKHTG (SEQ ID NO: 13),
WAPVLDFAPPGASAYGSL (SEQ ID NO: 14),
CWAPVLDFAPPGASAYGSL (SEQ ID NO: 15) and
WAPVLDFAPPGASAYGSLC (SEQ ID NO: 16), or a pharmaceutically acceptable salt thereof.

7. The method according to any one of claims 1 to 6, wherein the pharmaceutical composition comprises a pharmaceutically acceptable carrier.

8. The method according to any one of claims 1 to 7, wherein the body fluid is one or more selected from blood and spinal fluid.

9. The method according to any one of claims 1 to 8, further comprising: determining the mRNA expression level of WT1 gene by using a sample collected from the subject; and providing an indication that the subject is a potential subject with benefiting from the pharmaceutical composition in the case that the mRNA expression level of WT1 gene is less than a reference value or the reference value or less.

10. The method according to any one of claims 1 to 9, further comprising:
detecting a WT1 antigen peptide-specific CD8 T cell by using a sample collected from the subject which has been given the pharmaceutical composition or the peptide or the pharmaceutically acceptable salt thereof according to any one of claims 1 to 8; and
providing an indication that the subject is a potential subject with benefiting from the pharmaceutical composition in the case that the WT1 antigen peptide-specific CD8 T cell has increased as compared with a sample collected from the subject before administration.

11. The method according to claim 10, wherein detecting a WT1 antigen peptide-specific CD8 T cell is carried out by reacting a complex of a WT1 peptide and a HLA molecule with the sample, and examining the presence or cell number of a WT1 antigen peptide-specific CD8 T cell recognizing the complex contained in the sample.

12. The method according to claim 11, wherein the complex of a WT1 peptide and an HLA molecule is in the form of a tetramer.

13. The method according to claim 11 or 12, wherein the HLA molecule is compatible with HLA of the subject.

14. The method according to any one of claims 10 to 13, wherein detecting a WT1 antigen peptide-specific CD8 T cell comprises analysis by a flow cytometry method.

15. The method according to any one of claims 1 to 14, further comprising providing an indication that the subject is a potential subject with benefiting from the pharmaceutical composition in the case that delayed type hypersensitivity reaction has been detected in the subject which has been given a plurality of times the pharmaceutical composition or the peptide or the pharmaceutically acceptable salt thereof according to any one of claims 1 to 8.

16. The method according to claim 15, further comprising comparing reaction at an administration site of the pharmaceutical composition or the peptide or the pharmaceutically acceptable salt thereof according to any one of claims 1 to 8 in the subject with reaction at a non-administration site of the pharmaceutical composition or the peptide or the pharmaceutically acceptable salt thereof in the subject, and providing an indication that the subject is a potential subject with benefiting from the pharmaceutical composition in the case that the difference between the reaction at an administration site and the reaction at a non-administration site is a reference value or more.

## Patentansprüche

1. Verfahren zur Auswahl eines potenziellen Individuums, das von einer pharmazeutischen Zusammensetzung zur Behandlung oder Vorbeugung von Krebs profitiert, umfassend:
Bestimmen des Vorhandenseins oder Nichtvorhandenseins einer Mutation im Tumorprotein-p53-(TP53)-Gen und im BCL6-Co-Repressor-(BCOR)-Gen durch Verwenden einer Probe von Körperflüssigkeit, die von dem Individuum gesammelt wurde; und
Bereitstellen einer Angabe, dass das Individuum ein potenzielles Individuum ist, das von einer pharmazeutischen Zusammensetzung profitiert, wenn TP53-Wildtyp und BCOR-Wildtyp vorliegen, wobei die pharmazeutische Zusammensetzung ein Peptid umfasst, das eine Aminosäuresequenz umfasst, die ausgewählt ist aus der Gruppe bestehend aus RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7), VLDFAPPGA (SEQ ID NO: 9), CMTWNQMNL (SEQ ID NO: 3) und CYTWNQMNL (SEQ ID NO: 4) oder ein pharmazeutisch akzeptables Salz davon,
der Krebs ausgewählt ist aus der Gruppe bestehend aus akuter myeloischer Leukämie (AML), myelodysplastischem Syndrom (MDS), myeloproliferativer Neoplasie (MPN), chronischer myeloischer Leukämie (CML), chronischer myelomonozytärer Leukämie (CMML) und juveniler myelomonozytärer Leukämie (JMML),
der TP53-Wildtyp sich auf den Fall bezieht, dass keine Mutation im TP53-Gen vorhanden ist, oder den Fall, dass das TP53-Gen, selbst wenn eine Mutation vorhanden ist, keine ursprüngliche Funktion verliert oder nicht mit einer Abnormalität einhergeht, und
der BCOR-Wildtyp sich auf den Fall bezieht, dass keine Mutation im BCOR-Gen vorhanden ist, oder den Fall, dass das BCOR-Gen, selbst wenn eine Mutation vorhanden ist, keine ursprüngliche Funktion verliert oder nicht mit einer Abnormalität einhergeht.

2. Verfahren nach Anspruch 1, wobei die pharmazeutische Zusammensetzung umfasst:
ein Peptid, das eine Aminosäuresequenz umfasst, die ausgewählt ist aus der Gruppe bestehend aus RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7) und VLDFAPPGA (SEQ ID NO: 9), und
ein Peptid, das eine Aminosäuresequenz umfasst, die ausgewählt ist aus der Gruppe bestehend aus CMTWNQMNL (SEQ ID NO: 3) und CYTWNQMNL (SEQ ID NO: 4) oder ein pharmazeutisch akzeptables Salz davon.

3. Verfahren nach Anspruch 1 oder 2, wobei die pharmazeutische Zusammensetzung eine durch die Formel (1) dargestellte Verbindung umfasst: 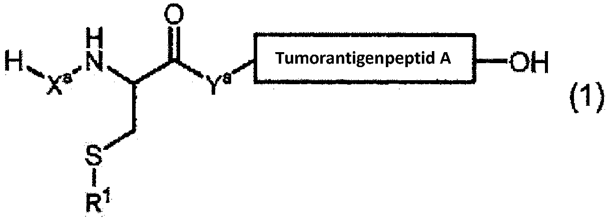 wobei X^{a} und Y^{a} eine Einfachbindung darstellen, Tumorantigenpeptid A ein Peptid darstellt, das aus einer beliebigen Aminosäuresequenz besteht, die aus den folgenden Aminosäuresequenzen ausgewählt ist:
RMFPNAPYL (SEQ ID NO: 2), YMFPNAPYL (SEQ ID NO: 8), ALLPAVPSL (SEQ ID NO: 5), SLGEQQYSV (SEQ ID NO: 6), RVPGVAPTL (SEQ ID NO: 7) und VLDFAPPGA (SEQ ID NO: 9),
die Aminogruppe der N-terminalen Aminosäure des Tumorantigenpeptids A an Y^{a} in der Formel (1) gebunden ist, die Carbonylgruppe der C-terminalen Aminosäure des Tumorantigenpeptids A an die Hydroxygruppe in der Formel (1) gebunden ist,
R¹ ein Wasserstoffatom oder Tumorantigenpeptid B darstellt,
das Tumorantigenpeptid B sich in der Sequenz vom Tumorantigenpeptid A unterscheidet und ein Peptid darstellt, das aus einer beliebigen Aminosäuresequenz besteht, die aus den folgenden Aminosäuresequenzen ausgewählt ist: CMTWNQMNL (SEQ ID NO: 3) und CYTWNQMNL (SEQ ID NO: 4), und die Thioethergruppe des Cysteinrests des Tumorantigenpeptids B an die Thioethergruppe in der Formel (1) gebunden ist, oder ein pharmazeutisch akzeptables Salz davon.

4. Verfahren nach Anspruch 3, wobei die durch die Formel (1) dargestellte Verbindung eine durch die Formel (2) dargestellte Verbindung ist: 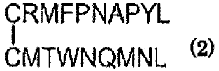
wobei die Bindung zwischen C und C eine Disulfidbindung darstellt,
oder ein pharmazeutisch akzeptables Salz davon.

5. Verfahren nach Anspruch 3, wobei die durch die Formel (1) dargestellte Verbindung eine durch die Formel (3) dargestellte Verbindung ist: 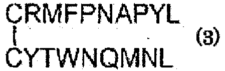
wobei die Bindung zwischen C und C eine Disulfidbindung darstellt,
oder ein pharmazeutisch akzeptables Salz davon.

6. Verfahren nach einem der Ansprüche 1 bis 5, wobei die pharmazeutische Zusammensetzung ferner ein Peptid umfasst, das eine Aminosäuresequenz umfasst, die ausgewählt ist aus der Gruppe bestehend aus
CNKRYFKLSHLQMHSRK (SEQ ID NO: 11),
CNKRYFKLSHLQMHSRKH (SEQ ID NO: 12),
CNKRYFKLSHLQMHSRKHTG (SEQ ID NO: 13),
WAPVLDFAPPGASAYGSL (SEQ ID NO: 14),
CWAPVLDFAPPGASAYGSL (SEQ ID NO: 15) und
WAPVLDFAPPGASAYGSLC (SEQ ID NO: 16), oder ein pharmazeutisch akzeptables Salz davon.

7. Verfahren nach einem der Ansprüche 1 bis 6, wobei die pharmazeutische Zusammensetzung einen pharmazeutisch akzeptablen Träger umfasst.

8. Verfahren nach einem der Ansprüche 1 bis 7, wobei die Körperflüssigkeit eine oder mehrere ist, ausgewählt aus Blut und Rückenmarksflüssigkeit.

9. Verfahren nach einem der Ansprüche 1 bis 8, ferner umfassend:
Bestimmen des mRNA-Expressionsniveaus des WT1-Gens durch Verwenden einer von dem Individuum gesammelten Probe; und Bereitstellen einer Angabe, dass das Individuum ein potenzielles Individuum ist, das von einer pharmazeutischen Zusammensetzung profitiert, wenn das mRNA-Expressionsniveau des WT1-Gens kleiner als ein Referenzwert ist oder dem Referenzwert oder weniger entspricht.

10. Verfahren nach einem der Ansprüche 1 bis 9, ferner umfassend:
Nachweisen einer WT1-Antigenpeptid-spezifischen CD8-T-Zelle durch Verwenden einer von dem Individuum gesammelten Probe, dem die pharmazeutische Zusammensetzung oder das Peptid oder das pharmazeutisch akzeptable Salz davon nach einem der Ansprüche 1 bis 8 verabreicht wurde; und
Bereitstellen einer Angabe, dass das Individuum ein potenzielles Individuum ist, das von einer pharmazeutischen Zusammensetzung profitiert, wenn die WT1-Antigenpeptid-spezifische CD8-T-Zelle im Vergleich zu einer vor der Verabreichung von dem Individuum gesammelten Probe zugenommen hat.

11. Verfahren nach Anspruch 10, wobei das Nachweisen einer WT1-Antigenpeptid-spezifischen CD8-T-Zelle durchgeführt wird, indem ein Komplex aus einem WT1-Peptid und einem HLA-Molekül mit der Probe umgesetzt wird und das Vorhandensein oder die Zellzahl einer WT1-Antigenpeptid-spezifischen CD8-T-Zelle, die den in der Probe enthaltenen Komplex erkennt, untersucht wird.

12. Verfahren nach Anspruch 11, wobei der Komplex aus einem WT1-Peptid und einem HLA-Molekül in Form eines Tetramers vorliegt.

13. Verfahren nach Anspruch 11 oder 12, wobei das HLA-Molekül mit dem HLA des Individuums kompatibel ist.

14. Verfahren nach einem der Ansprüche 10 bis 13, wobei das Nachweisen einer WT1-Antigenpeptid-spezifischen CD8-T-Zelle eine Analyse durch ein Durchflusszytometrieverfahren umfasst.

15. Verfahren nach einem der Ansprüche 1 bis 14, ferner umfassend das Bereitstellen einer Angabe, dass das Individuum ein potenzielles Individuum ist, das von einer pharmazeutischen Zusammensetzung profitiert, wenn eine Spättyp-Überempfindlichkeitsreaktion bei dem Individuum nachgewiesen wurde, dem eine Vielzahl von Malen die pharmazeutische Zusammensetzung oder das Peptid oder das pharmazeutisch akzeptable Salz davon nach einem der Ansprüche 1 bis 8 verabreicht wurde.

16. Verfahren nach Anspruch 15, ferner umfassend das Vergleichen der Reaktion an einer Verabreichungsstelle der pharmazeutischen Zusammensetzung oder des Peptids oder des pharmazeutisch akzeptablen Salzes davon nach einem der Ansprüche 1 bis 8 bei dem Individuum mit einer Reaktion an einer Nicht-Verabreichungsstelle der pharmazeutischen Zusammensetzung oder des Peptids oder des pharmazeutisch akzeptablen Salzes davon bei dem Individuum, und Bereitstellen einer Angabe, dass das Individuum ein potenzielles Individuum ist, das von einer pharmazeutischen Zusammensetzung profitiert, wenn der Unterschied zwischen der Reaktion an einer Verabreichungsstelle und der Reaktion an einer Nicht-Verabreichungsstelle einem Referenzwert oder mehr entspricht.

## Revendications

1. Méthode pour sélectionner un sujet potentiel pouvant tirer profit d'une composition pharmaceutique pour traiter ou prévenir un cancer, comprenant :
la détermination de la présence ou de l'absence d'une mutation dans le gène de protéine tumorale p53 (TP53) et le gène de corépresseur BCL6 (BCOR) par utilisation d'un échantillon de fluide corporel collecté auprès du sujet ; et
la fourniture d'une indication que le sujet est un sujet potentiel pouvant tirer profit de la composition pharmaceutique dans le cas du type sauvage de TP53 et du type sauvage de BCOR, dans laquelle la composition pharmaceutique comprend un peptide comprenant une séquence d'acides aminés choisie dans le groupe constitué par RMFPNAPYL (SEQ ID NO : 2), YMFPNAPYL (SEQ ID NO : 8), ALLPAVPSL (SEQ ID NO : 5), SLGEQQYSV (SEQ ID NO : 6), RVPGVAPTL (SEQ ID NO : 7), VLDFAPPGA (SEQ ID NO : 9), CMTWNQMNL (SEQ ID NO : 3) et CYTWNQMNL (SEQ ID NO : 4), ou un de ses sels pharmaceutiquement acceptables,
le cancer est choisi dans le groupe constitué par une leucémie aiguë myéloïde (LAM), un syndrome myélodysplasique (SMD), une néoplasie myéloproliférative (NMP), une leucémie myéloïde chronique (LMC), une leucémie myélomonocytaire chronique (LMMC) et une leucémie myélomonocytaire juvénile (LMMJ),
le type sauvage de TP53 se réfère au cas où aucune mutation n'est présente dans le gène de TP53, ou au cas où le gène de TP53, même si une mutation est présente, ne perd pas de fonction originelle ou ne s'accompagne pas d'une anomalie, et
le type sauvage de BCOR se réfère au cas ou aucune mutation n'est présente dans le gène de BCOR, ou au cas où le gène de BCOR, même si une mutation est présente, ne perd pas de fonction originelle ou ne s'accompagne pas d'une anomalie.

2. Méthode selon la revendication 1, dans laquelle la composition pharmaceutique comprend
un peptide comprenant une séquence d'acides aminés choisie dans le groupe constitué par RMFPNAPYL (SEQ ID NO : 2), YMFPNAPYL (SEQ ID NO : 8), ALLPAVPSL (SEQ ID NO : 5), SLGEQQYSV (SEQ ID NO : 6), RVPGVAPTL (SEQ ID NO : 7) et VLDFAPPGA (SEQ ID NO : 9), et
un peptide comprenant une séquence d'acides aminés choisie dans le groupe constitué par CMTWNQMNL (SEQ ID NO : 3) et CYTWNQMNL (SEQ ID NO : 4), ou un de ses sels pharmaceutiquement acceptables.

3. Méthode selon la revendication 1 ou 2, dans laquelle la composition pharmaceutique comprend un composé représenté par la formule (1) : 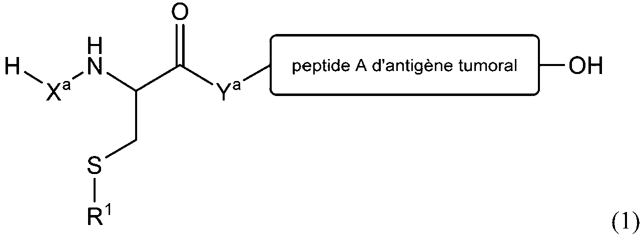 dans laquelle X^{a} et Y^{a} représentent une liaison simple, "peptide A d'antigène tumoral" représente un peptide consistant en n'importe quelle séquence d'acides aminés choisie parmi les séquences d'acides aminés suivantes :
RMFPNAPYL (SEQ ID NO : 2), YMFPNAPYL (SEQ ID NO : 8), ALLPAVPSL (SEQ ID NO : 5), SLGEQQYSV (SEQ ID NO : 6), RVPGVAPTL (SEQ ID NO : 7) et VLDFAPPGA (SEQ ID NO : 9),
le groupe amino de l'acide aminé N-terminal du peptide A d'antigène tumoral est lié à Y^{a} dans la formule (1), le groupe carbonyle de l'acide aminé C-terminal du peptide A d'antigène tumoral est lié au groupe hydroxy dans la formule (1),
R¹ représente un atome d'hydrogène ou un peptide d'antigène tumoral B,
le peptide d'antigène tumoral B a une séquence qui diffère de celle du peptide d'antigène tumoral A, et représente un peptide consistant en n'importe quelle séquence d'acides aminés choisie parmi les séquences d'acides aminés suivantes : CMTWNQMNL (SEQ ID NO : 3) et CYTWNQMNL (SEQ ID NO : 4), et le groupe thioéther du résidu cystéine du peptide d'antigène tumoral B est lié au groupe thioéther dans la formule (1), ou un de ses sels pharmaceutiquement acceptables.

4. Méthode selon la revendication 3, dans laquelle le composé représenté par la formule (1) est un composé représenté par la formule (2) : 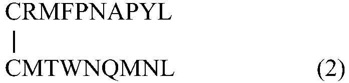
dans laquelle la liaison entre C et C représente une liaison disulfure,
ou un de ses sels pharmaceutiquement acceptables.

5. Méthode selon la revendication 3, dans laquelle le composé représenté par la formule (1) est un composé représenté par la formule (3) : 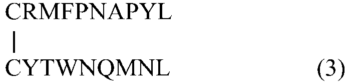
dans laquelle la liaison entre C et C représente une liaison disulfure,
ou un de ses sels pharmaceutiquement acceptables.

6. Méthode selon l'une quelconque des revendications 1 à 5, dans laquelle la composition pharmaceutique comprend en outre un peptide comprenant une séquence d'acides aminés choisie dans le groupe constitué par
CNKRYFKLSHLQMHSRK (SEQ ID NO : 11),
CNKRYFKLSHLQMHSRKH (SEQ ID NO : 12),
CNKRYFKLSHLQMHSRKHTG (SEQ ID NO : 13),
WAPVLDFAPPGASAYGSL (SEQ ID NO : 14),
CWAPVLDFAPPGASAYGSL (SEQ ID NO : 15) et
WAPVLDFAPPGASAYGSLC (SEQ ID NO: 16),
ou un de ses sels pharmaceutiquement acceptables.

7. Méthode selon l'une quelconque des revendications 1 à 6, dans laquelle la composition pharmaceutique comprend un véhicule pharmaceutiquement acceptable.

8. Méthode selon l'une quelconque des revendications 1 à 7, dans laquelle le fluide corporel est un ou plusieurs choisis parmi le sang et le liquide céphalo-rachidien.

9. Méthode selon l'une quelconque des revendications 1 à 8, comprenant en outre : la détermination du niveau d'expression d'ARNm du gène de WT1 par utilisation d'un échantillon collecté auprès du sujet ; et la fourniture d'une indication que le sujet est un sujet potentiel pouvant tirer profit de la composition pharmaceutique dans le cas où le niveau d'expression d'ARNm du gène de WT1 est inférieur à une valeur de référence, ou correspond à la valeur de référence ou à une valeur moindre.

10. Méthode selon l'une quelconque des revendications 1 à 9, comprenant en outre :
la détection d'une cellule T CD8 spécifique d'un peptide antigénique de WT1 par utilisation d'un échantillon collecté auprès du sujet auquel a été administré la composition pharmaceutique ou le peptide ou son sel pharmaceutiquement acceptable selon l'une quelconque des revendications 1 à 8 ; et
la fourniture d'une indication que le sujet est un sujet potentiel pouvant tirer profit de la composition pharmaceutique dans le cas où la cellule T CD8 spécifique d'un peptide antigénique de WT1 a augmenté en comparaison avec un échantillon collecté auprès du sujet avant l'administration.

11. Méthode selon la revendication 10, dans laquelle la détection d'une cellule T CD8 spécifique d'un peptide antigénique de WT1 est réalisée par réaction d'un complexe d'un peptide de WT1 et d'une molécule de HLA avec l'échantillon, et examen de la présence ou du nombre cellulaire d'une cellule T CD8 spécifique d'un peptide antigénique de WT1 reconnaissant le complexe contenu dans l'échantillon.

12. Méthode selon la revendication 11, dans laquelle le complexe d'un peptide de WT1 et d'une molécule de HLA est sous la forme d'un tétramère.

13. Méthode selon la revendication 11 ou 12, dans laquelle la molécule de HLA est compatible avec le HLA du sujet.

14. Méthode selon l'une quelconque des revendications 10 à 13, dans laquelle la détection d'une cellule T CD8 spécifique d'un peptide antigénique de WT1 comprend une analyse par une méthode de cytométrie en flux.

15. Méthode selon l'une quelconque des revendications 1 à 14, comprenant en outre la fourniture d'une indication que le sujet est un sujet potentiel pouvant tirer profit de la composition pharmaceutique dans le cas où une réaction d'hypersensibilité de type retardé a été détectée chez le sujet qui a reçu plusieurs fois la composition pharmaceutique ou le peptide ou son sel pharmaceutiquement acceptable selon l'une quelconque des revendications 1 à 8.

16. Méthode selon la revendication 15, comprenant en outre la comparaison de la réaction au niveau d'un site d'administration au sujet de la composition pharmaceutique ou du peptide ou de son sel pharmaceutiquement acceptable selon l'une quelconque des revendications 1 à 8 avec la réaction au niveau d'un site de non administration au sujet de la composition pharmaceutique ou du peptide ou de son sel pharmaceutiquement acceptable, et la fourniture d'une indication que le sujet est un sujet potentiel pouvant tirer profit de la composition pharmaceutique dans le cas où la différence entre la réaction au niveau d'un site d'administration et la réaction au niveau d'un site de non administration correspond à une valeur de référence ou est supérieure à cette valeur.
